(19) **Europäisches Patentamt / European Patent Office / Office européen des brevets**

(11) **EP 4 616 199 B1**

(12) **EUROPEAN PATENT SPECIFICATION**

(45) Date of publication and mention
of the grant of the patent:
**20.05.2026 Bulletin 2026/21**

(21) Application number: **23801769.3**

(22) Date of filing: **07.11.2023**

(51) International Patent Classification (IPC):
***G01N 33/68*** *(2006.01)*

(52) Cooperative Patent Classification (CPC):
**G01N 33/689; G01N 2800/364; G01N 2800/52**

(86) International application number:
**PCT/EP2023/080988**

(87) International publication number:
**WO 2024/100036 (16.05.2024 Gazette 2024/20)**

(54) **IMPROVED METHOD FOR THE SCREENING, DIAGNOSIS OR MONITORING OF ENDOMETRIOSIS, KIT AND USES THEREOF**

VERBESSERTES VERFAHREN ZUM SCREENING, ZUR DIAGNOSE ODER ZUR ÜBERWACHUNG VON ENDOMETRIOSE, KIT UND VERWENDUNGEN DAVON

PROCÉDÉ AMÉLIORÉ DE DÉPISTAGE, DE DIAGNOSTIC OU DE SURVEILLANCE DE L'ENDOMÉTRIOSE, KIT ET UTILISATIONS DE CELUI-CI

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.11.2022 EP 22383069**

(43) Date of publication of application:
**17.09.2025 Bulletin 2025/38**

(73) Proprietor: **Chemo Research, S.L.**
**28050 Madrid (ES)**

(72) Inventors:
• **HERRANZ BLANCO, Bárbara**
**28050 Madrid (ES)**
• **COLLI, Enrico**
**28050 Madrid (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**US-A1- 2018 067 102**

• **WESSELS JOCELYN M ET AL: "Assessing brain-derived neurotrophic factor as a novel clinical marker of endometriosis", FERTILITY AND STERILITY, ELSEVIER, AMSTERDAM, NL, vol. 105, no. 1, 25 September 2015 (2015-09-25), pages 119, XP029375287, ISSN: 0015-0282, DOI: 10.1016/J.FERTNSTERT.2015.09.003**
• **PAL ARIJIT ET AL: "Development of an impedimetric immunosensor for machine learning-based detection of endometriosis: A proof of concept", SENSORS AND ACTUATORS B: CHEMICAL, ELSEVIER BV, NL, vol. 346, 29 July 2021 (2021-07-29), XP086759529, ISSN: 0925-4005, [retrieved on 20210729], DOI: 10.1016/J.SNB.2021.130460**

Remarks:
The complete document including Reference Table(s) and the Sequence Listing(s) can be downloaded from the EPO website

**Description**

**FIELD OF THE INVENTION**

The present invention relates to the field of diagnosis of endometriosis. Specifically, it relates to an improved non-invasive method for the screening, diagnosis or monitoring of endometriosis in a subject, preferably a female subject, comprising the determination of the protein levels of a group of biomarkers comprising or consisting of brain-derived neurotrophic factor (BDNF) and/or cancer antigen 125 (CA125) in a biological sample, optionally in combination with variables related to the subject's history and/or clinical characteristics. It further relates to the use of kit for determining the protein levels of BDNF and/or CA125 and the use of the kit in the methods of the invention.

**BACKGROUND OF THE INVENTION**

Endometriosis is a painful, inflammatory condition characterized by endometrial-like tissue outside the uterus (Johnson, 2013; Zondervan, 2018 and 2020). These lesions cause a chronic, inflammatory reaction, which can lead to the generation of scar tissue and adhesions. The disease is estimated to affect 10% of women of reproductive age, which extrapolates to approximately 190 million women worldwide (Vigano, 2004). Clinical symptoms include chronic pelvic pain, dysmenorrhea, and infertility (Vigano, 2004). Endometriosis may increase a woman's risk of cancer or autoimmune disorders (Somigliana, 2006; Sinaii, 2002) as well. Endometriotic lesions can occur at different locations, including the pelvic peritoneum and the ovary, or infiltrate pelvic structures below the peritoneal surface (deep endometriosis).

Diagnosis of endometriosis is difficult, often resulting in many years between the first appearance of symptoms and the final confirmation of the diagnosis. Diagnosis time ranges from 4 to 11 years (Agarwal, 2019). The clinical examination of symptomatic women does not reliably predict the presence of endometriosis and the current gold standard for diagnosing the disease is visual inspection with histologic confirmation of lesions by laparoscopy. However, due to its invasiveness, it is now only recommended in patients with negative imaging results and/or where empirical treatment was unsuccessful or inappropriate (The ESHRE guideline: endometriosis, 2022).

Indeed, due to the invasiveness required for diagnosis, many women with endometriosis receive suboptimal, or inappropriate care. Generally, women for whom there is high suspicion of endometriosis receive analgesics and hormonal medication without a prior definitive diagnosis (Dunselman, 2014). It is common practice for these patients to receive laparoscopy only if improvements from hormonal contraceptives are not observed in order to exclude or diagnose endometriosis. However, a response to hormonal treatment does not always indicate presence or absence of endometriosis (Ling, 1999; Jenkins, 2008). Therefore, women with other potentially serious estrogen dependent conditions may be misclassified as having endometriosis, and not receive necessary and effective treatments. Thus, presumptive treatment of symptomatic women can lead to misdiagnosis in women without endometriosis and contribute to a delay in diagnosis in women with the disease.

The development of novel non-invasive diagnostic tool relying on a panel of biomarkers associated with endometriosis is a promising approach for faster diagnosis, appropriate treatment, and triaging potential surgical patients (Ahn SH, et al., 2017).

Multiple biomarkers (Nisenblat, 2016a, 2016b) have been studied as screening and triage tests for endometriosis. However, none of them have been implemented routinely in clinical practice (The ESHRE guideline: endometriosis, 2022).

WO 2015051451 (MCMASTER UNIVERSITY, CA) discloses a method for diagnosing or monitoring endometriosis comprising determining the expression levels of BDNF and its receptor Ntrk2 in peripheral blood and diagnosing the mammal with endometriosis when the BDNF level and Ntrk2 level are both elevated by at least 10% as compared with the control levels.

US 20180067102 provides methods for diagnosing or monitoring endometriosis in a mammal. It discloses a method for diagnosing endometriosis comprising determining the expression levels of BDNF and glycodelin and optionally zinc-alpha-2-glycoprotein (ZAG), in a biological sample from the mammal, and determining that the mammal has endometriosis when the biomarker expression levels in the sample are elevated. It discloses the testing of a panel of biomarkers: BDNF and glycodelin were found to be superior to both emerging (SERPINE2 and ZAG) and classical markers (CA-125, ZAG, VEGF, IL-6, IL-Iß, RANTES, sICAM-1, and leptin) as single non-invasive markers for the diagnosis of endometriosis in the study population. Furthermore, it discloses that the combination of BDNF, glycodelin and ZAG in a panel produced a sensitivity and specificity that was superior to either marker alone.

Despite extensive research efforts, a reliable non-invasive test for the diagnosis or monitoring of endometriosis is still to be found.

## SUMMARY OF THE INVENTION

The present inventors conducted a new evaluation of a panel of biomarkers including ZAG, glycodelin, BDNF and CA125 in serum and plasma for the diagnosis of endometriosis in a European female population, more specifically from the UK and Italy.

As shown in Example 1, Table 3, it was found a high correlation (0.999) of the concentration levels of CA125 between plasma and serum, indicating that the measurement of CA125 levels was not affected by specimen type. However, such correlation between the concentration levels in the two types of biological samples was not observed for BDNF which presented a very low correlation coefficient (0.105). Accordingly, the diagnostic performance of BDNF was found to be significantly higher when measured in serum than in plasma.

Indeed, CA125 (in plasma or serum) and BDNF in serum were found to have a statistically significant association with endometriosis (Example 1, Table 4) and to be useful predictive biomarkers (Example 1, Table 5) in the population of study.

Besides, despite WO 2015051451 and US 20180067102 having found a statistically significant association between the concentration levels in plasma of glycodelin and ZAG and presence of endometriosis, such results could not be reproduced in the present study. where ZAG and glycodelin were found to have worse predictive power. Thus, unexpectedly the combination of CA125 and serum BDNF, was found to be the most relevant biomarkers for the diagnosis of endometriosis .

Moreover, the inventors further identified a series of parameters related to the history and/or clinical characteristics of the subjects which were found to also be associated with endometriosis (Example 3), which enabled to increase the predictive diagnostic power of the serum CA125 and BDNF biomarkers (Example 4).

Accordingly, the first aspect of the invention relates to a method for the screening, diagnosis or monitoring of endometriosis or the presence of endometriosis type lesions, or for predicting the presence of endometriosis type lesions in a female subject, wherein said method comprises:

a) determining the protein levels of a biomarker or a group of biomarkers in a blood, plasma or serum sample isolated from the subject, wherein said biomarkers comprise or consist of:

i. cancer antigen 125 (CA125), and
ii. brain-derived neurotrophic factor (BDNF); and

b) optionally, calculating a score from the protein levels determined in step a), optionally in combination with other gene or protein markers, biochemical parameters and/or variables related to the subject's history and/or clinical characteristics;

c) comparing the protein levels of CA125 and BDNF in the subject's sample as determined in step a) with a corresponding reference value; or comparing the score obtained in b) with a reference value; wherein when the CA125 and BDNF protein levels in a), or the score in b), are increased with respect to the respective reference value is indicative of endometriosis or presence of endometriosis type lesions.

In a second aspect, the present invention relates to a computer implemented method, wherein the method is as claimed.

A third aspect of the invention pertains to a computer program which causes a computer rry out the claimed computer-implemented method.

In a fourth aspect, the present invention relates to a data-processing apparatus comprising means for carrying out steps ii) and/or iii) of the claimed computer-implemented method.

In an embodiment, the method of the invention further comprises conducting a confirmatory test, such as a histologic confirmation after laparoscopy, in a subject suspected of having endometriosis or endometriosis-like lesions, wherein said subject has been selected as having endometriosis or endometriosis-like lesions by a method as described herein above.

In a seventh aspect, the invention pertains to the use of a kit for determining the levels of the biomarkers as described herein in a biological sample as described herein (preferably a serum sample) isolated from a subject.

In an eight aspect, the invention relates to the use of a kit as described above in any of the methods as described herein, such as a method for the screening, diagnosing or monitoring of endometriosis or endometriosis-like lesions in a subject; a method of predicting the degree of endometriosis or for classifying a subject according to the degree or phenotype of endometriosis as described herein; a method for subject selection or a method for subject selection and performing an exploratory test (such as a as a histologic confirmation after laparoscopy) as described herein above .

## BRIEF DESCRIPTION OF THE DRAWINGS

FIG.1: CA125 protein levels in plasma for cases and controls
FIG.2: CA125 protein levels in serum for cases and controls.
FIG.3: Glycodelin protein levels in plasma for cases and controls.
FIG.4: Glycodelin protein levels in serum for cases and controls.
FIG.5: Total BDNF levels in plasma for cases and controls.
FIG.6: Total BDNF levels in serum for cases and controls.
FIG.7: ZAG protein levels in plasma for cases and controls.
FIG.8: ZAG protein levels in serum for cases and controls.
FIG.9: Degree of association of serum CA125 (IU/mL) vs plasma CA125 (IU/mL).
FIG.10: Degree of association of serum Glycodelin (ng/mL) vs plasma Glycodelin (ng/mL).
FIG.11: Degree of association of serum Total BDNF (pg/mL) vs plasma Total BDNF (pg/mL).
FIG.12: Degree of association of serum ZAG (ng/mL) vs plasma ZAG (ng/mL).
FIG.13: ROC Curves of CA125 and BDNF.
FIG.14: Access numbers of mRNA and protein sequences of BDNF transcripts in the NCBI database
FIG. 15: Access numbers of mRNA and protein sequences of CA125 transcripts in the NCBI database
FIG. 16: human CA125 protein sequence (SEQ ID NO: 5)

## DETAILED DESCRIPTION OF THE INVENTION

### *Definitions*

The terms "subject", or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a female human being of any age or race. In some embodiments, it is a woman of reproductive age, wherein the expression woman of reproductive age refers to a pre-menopause woman, preferably between 15 and 49 years.

As used herein the term "endometriosis" refers to a chronic, estrogen-dependent inflammatory condition that is characterized by the formation of endometriotic lesions outside the uterus including the ovaries and other pelvic structures. Symptoms are influenced by ovarian hormones and lesions cause intense inflammation in the pelvic cavity. They become vascularized and sensory nerve fibers infiltrate them. Ectopic endometrial cells and the local inflammatory environment activate nerve fibers in lesions, establishing communication with the central nervous system and causing pain. Lesions, like eutopic endometrium, exhibit cyclical bleeding into the pelvic cavity in response to ovarian hormones, which promotes inflammation. Main symptoms include pain and infertility. The latest recommendations on the diagnosis, management and treatment of endometriosis are provided in the ESHRE guideline: endometriosis, 2022.

All phenotypes of endometriosis are encompassed herein. In some embodiments, endometriosis phenotypes are defined according to the location of the endometriosis type lesions, including superficial, peritoneal lesions (SUP), ovarian endometriomas (OMA) and deep infiltrating endometriosis (DIE) (see Chapron et al. Rethinking mechanisms, diagnosis and management of endometriosis, Nat Rev Endocrinol. 2019 Nov;15(11):666-682). In some embodiments, endometriosis phenotypes are defined according to any of the known classifications, such as those described herein below.

The current classifications of disease (rAFS/rASRM) are based on lesion size, location, extent of lesion infiltration into tissue, and/or the presence of adhesions. The stages are numbered from I ("minimal") to IV ("severe") (see Lee SY, Koo YJ, Lee DH. Classification of endometriosis. Yeungnam Univ J Med. 2021 Jan;38(1):10-18 i).

Common pelvic sites of endometriosis include the ovary and ovarian fossa, uterosacral ligaments, pouch of Douglas, and bladder. Endometriosis is staged at the time of surgery; many classifications have been proposed.

**1/ The revised American Society for Reproductive Medicine scoring system (ASRM) is** commonly used. Stages - Components of the ASRM scoring system (Revised American Society for Reproductive Medicine classification of endometriosis: 1996. Fertil Steril. 1997 May;67(5):817-21; Lee et al. 2021), include:

- Stage I - Minimal disease is characterized by isolated implants and no significant adhesions.

- Stage II - Mild endometriosis consists of superficial implants that are less than 5 cm in aggregate and are scattered on the peritoneum and ovaries. No significant adhesions are present.

- Stage III - Moderate disease exhibits multiple implants, both superficial and deeply invasive. Peritubal and periovarian adhesions may be evident.

- Stage IV - Severe disease is characterized by multiple superficial and deep implants, including large ovarian endometriomas. Filmy and dense adhesions are usually present.

**2/ The Enzian classification** (Keckstein J, et al. 2021)

The ENZIAN classification was created as a supplement to the rASRM score to provide a morphologically descriptive classification of Deep infiltrating endometriosis (DIE) that takes retroperitoneal structures into account. Retroperitoneal structures are classified into three compartments in this classification:

- Compartment A: vagina, recto-vaginal septum;
- Compartment B: uterosacral ligaments to the pelvic wall (BB: bilateral involvement);
- Compartment C: rectum and sigmoid colon.

Disease severity is classified as:

- Grade 1: invasion 3 cm Deep endometriosis invasion beyond the lesser pelvis and invasion of organs are recorded separately:
- FA: adenomyosis;
- FB: bladder invasion;
- FU: intrinsic ureteral endometriosis;
- FI: bowel disease cranial to the sigmoid colon;
- F0: other locations.

The prefix "F" stands for "far" or "foreign", referring to distant retroperitoneal structures. The ENZIAN classification nomenclature, which is similar to the TNM (Tumor, Lymph Nodes, Metastasis) staging system used in oncologic diseases, is the following: A0-3 B0-3 C0-3 FA, FB, FU, FI, FO. Distant locations are only stated when present. When more than one focus is present in each compartment, only the largest is evaluated (Capezzuoli et al., 2020).

The term "endometriosis type lesions" or "endometriosis-like lesions" is used herein interchangeably. Endometriosis type lesions refers to the growth of endometrial-like tissue outside the uterus. Endometriosis phenotypes can be defined according to the location of the endometriosis type lesions, including superficial, peritoneal lesions (SUP), ovarian endometrioma (OMA), and deep infiltrating endometriosis (DIE). In rare cases, endometriosis type lesions have also been found outside the pelvis.

The term "screening" is understood herein as the examination or testing of a group of asymptomatic individuals pertaining to the general population or having one or more risk factors with the objective of discriminating healthy individuals from those who have or are suspected of having a disease. A method of screening is generally used for the "early detection" of a disease. The expression "early detection" refers to detection before the presence of clinical signs.

The term "diagnosis", as used herein, refers both to the process of attempting to determine and/or identify a possible disease in a subject, i.e. the diagnostic procedure, and to the opinion reached by this process, i.e. the diagnostic opinion. In particular, the diagnosis to determine a disease in a subject, relates to the capacity to identify and classify patients having said disease. A "diagnostic test" could be used to suggest or rule out the disease.

The term "monitoring" as used herein refers to determining the evolution of the disease and/or the efficacy of a therapy, for example determining whether there is a remission of the disease; or on the contrary whether there is disease progression or a relapse.

The term "Receiver Operating Characteristic (ROC) curves" as used herein refers to a graphical plot that illustrates the performance of a binary classifier system as its discrimination threshold is varied. The curve is created by plotting the true positive rate against the false positive rate at various threshold settings. The true positive rate is also known as sensitivity. The false positive rate is calculated as 1 - specificity. The ROC curve is thus a way of graphically displaying the true positive rate versus the false positive rate (sensitivity vs (1-specificity)) across a range of cut-offs and of selecting the optimal cut-off for clinical use. Accuracy expressed as the area under the ROC curve (AUC) provides a useful parameter for comparing test performance. An AUC approaching 1 indicates that the test is highly sensitive as well as highly specific whereas an AUC approaching 0.5 indicates that the test is neither sensitive nor specific. In general, a test is considered to be a suitable discriminator if the AUC is from 0.6 to 0.75, to have high discrimination capacity if the AUC is from 0.75 to 0.9 and to be an excellent discriminator if the AUC is from 0.9 to 1. For further details see for instance, (Zweig and Campbell 1993; Greiner, Pfeiffer, and Smith 2000).

As used herein a "therapeutically effective amount" refers to an amount effective, at dosages and for periods of time necessary to achieve the desired therapeutic result, such as one or more of the following therapeutic results, such as a significant delay of the onset or progression of the disease; or a significant reduction of the severity of one or more symptoms. A therapeutically effective amount is also typically one in which any toxic or detrimental effect of the active ingredient or pharmaceutical composition is outweighed by the therapeutically beneficial effects.

As used herein, "treatment", "treating" or "treat" refer to: (i) preventing or retarding a disease, disorder or condition from occurring in a subject which may be predisposed to the disease, disorder and/or condition but has not yet been diagnosed as having it; (ii) inhibiting the disease, disorder or condition, i.e., arresting or slowing down its development or progression; and/or (iii) relieving the disease, disorder or condition, i.e., causing regression of the disease, disorder and/or condition. In certain embodiments, such term refers to the amelioration or eradication of a disease or symptoms associated with a disease.

The term "affinity reagent" may refer to a ligand (e.g., antibody, peptide, protein, nucleic acid or small molecule) that selectively captures (binds to) a target molecule through specific molecular recognition, typically with a binding affinity in the nanomolar to sub-nanomolar range. For example, the affinity reagent may be an aptamer, antibody or antibody-mimetic.

The term "affinity" as used herein may refer to the equilibrium constant for the dissociation of an antigen with an antigen-binding molecule (KD), and is considered a measure for the binding strength between an antigenic determinant and an antigen-binding site on the antigen -binding molecule: the lesser the value of the KD, the stronger the binding strength between an antigenic determinant and the antigen-binding molecule (alternatively, the affinity can also be expressed as the association constant (KA), which is 1/KD). It will be clear to the skilled person that the dissociation constant may be the actual or apparent dissociation constant.

The term "aptamer" or "nucleic acid aptamer" as used herein may refer to an isolated or purified single-stranded nucleic acid (RNA or DNA) that binds with high specificity and affinity to a target through interactions other than Watson-Crick base pairing. An aptamer has a three dimensional structure that provides chemical contacts to specifically bind to a target. Unlike traditional nucleic acid binding, aptamer binding is not dependent upon a conserved linear base sequence, but rather a particular secondary or tertiary structure. That is, the nucleic acid sequences of aptamers are non-coding sequences. Any coding potential that an aptamer may possess is entirely fortuitous and plays no role whatsoever in the binding of an aptamer to a target. A typical minimized aptamer is 5-15 kDa in size (15-45 nucleotides), binds to a target with nanomolar to sub-nanomolar affinity, and discriminates against closely related targets (e.g., aptamers will typically not bind to other proteins from the same gene or functional family).

The term "antibody" as used herein may refer to an immunoglobulin or an antigen-binding fragment thereof. Unless otherwise specified, the term includes, but is not limited to, polyclonal, monoclonal, monospecific, multispecific, humanized, human, chimeric, synthetic, recombinant, hybrid, mutated, grafted, and *in vitro* generated antibodies. The antibody can include a constant region, or a portion thereof, such as the kappa, lambda, alpha, gamma, delta, epsilon and mu constant region genes. For example, heavy chain constant regions of the various isotypes can be used, including: $IgG_1$, $IgG_2$, $IgG_3$, $IgG_4$, IgM, $IgA_1$, $IgA_2$, IgD, and IgE. By way of example, the light chain constant region can be kappa or lambda. In certain embodiments, the term "antibody" may also refer to antibody derivatives, such as antibody-based fusion proteins or antibodies further modified to contain additional non-proteinaceous moieties, such as water-soluble polymers, e.g. polyethylene glycol (PEG).

The terms "antigen-binding domain" and "antigen-binding fragment" refer to a part of an antibody molecule that comprises amino acids responsible for the specific binding between antibody and antigen. For certain antigens, the antigen-binding domain or antigen-binding fragment may only bind to a part of the antigen. The part of the antigen that is specifically recognized and bound by the antibody is referred to as the "epitope" or "antigenic determinant." Antigen-binding domains and antigen-binding fragments include Fab; a $F(ab')_2$ fragment (a bivalent fragment having two Fab fragments linked by a disulfide bridge at the hinge region); a Fv fragment; a single chain Fv fragment (scFv); a Fd fragment (having the two $V_H$ and $C_H1$ domains); single domain antibodies (sdAbs; consisting of a single $V_H$ domain), and other antibody fragments that retain antigen-binding function. The Fab fragment has $V_H$-$C_H1$ and $V_L$-$C_L$ domains covalently linked by a disulfide bond between the constant regions. The $F_v$ fragment is smaller and has $V_H$ and $V_L$ domains non-covalently linked. The $scF_v$ contains a flexible polypeptide that links (1) the C-terminus of $V_H$ to the N-terminus of $V_L$, or (2) the C-terminus of $V_L$ to the N-terminus of $V_H$. The sdAbs include heavy chain antibodies naturally devoid of light chains and single-domain antibodies derived from conventional four chain antibodies. These antigen-binding domains and fragments are obtained using conventional techniques known to those with skill in the art, and are evaluated for function in the same manner as are intact immunoglobulins.

The term "recombinant antibody" as used herein refers to an antibody produced or expressed using a recombinant expression vector, where the expression vector comprises a nucleic acid encoding the recombinant antibody, such that introduction of the expression vector into an appropriate host cell results in the production or expression of the recombinant antibody. Recombinant antibodies may be chimeric or humanized antibodies, mono- or multispecific antibodies.

The term "an antibody mimetic" (AbM) as used herein refers to single-domain scaffolds, which have been engineered to bind therapeutic targets with affinity and specificity that match that of natural antibodies. Antibody mimetics have been developed utilizing an immunoglobulin-like fold, for example, fibronectin type III, NCAM and CTLA-4. Other mimetics scaffolds bearing no similarity to immunoglobulin folds have also been obtained. Non-limiting examples of said scaffolds are DARPins, anticalins, affibodies, adnectins, fynomers, etc. (see for instance, Weidle et al. Cancer Genomics & Proteomics. 2013, 10:1-18; Lofblom, J. et al., Curr. Opin. Biotechnol. 2011, 22: 843-848; Banta, S. et al., Annu. Rev. Biomed. Eng., 2010, 15: 93-113).

## *Detailed description*

### *Method for the screening, diagnosis or monitoring of endometriosis*

In some embodiments, the present invention relates to a method for the screening, diagnosis or monitoring of endometriosis or the presence of endometriosis type lesions, or for predicting the presence of endometriosis type lesions in a female subject, wherein said method comprises:

a) determining the protein levels of a biomarker or a group of biomarkers comprising or consisting of:

i. cancer antigen 125 (CA125) in a blood, plasma or serum sample isolated from the subject, and
ii. brain-derived neurotrophic factor (BDNF) in a serum sample isolated from the subject; and

b) optionally, calculating a score from the protein levels determined in step a), optionally in combination with other gene or protein markers, biochemical parameters and/or variables related to the subject's history and/or clinical characteristics;
c) comparing the protein levels of CA125 and BDNF in the subject's sample as determined in step a) with a corresponding reference value; or comparing the score obtained in b) with a reference value; wherein when the CA125 and BDNF protein levels in a), or the score in b), are increased with respect to the respective reference value is indicative of endometriosis or presence of endometriosis type lesions.

As defined herein above, endometriosis is a chronic, debilitating disease defined as endometrial-like glands or stroma outside of the uterine cavity that leads to an oestrogen-dependent chronic inflammatory state, associated with dysmenorrhea (non-menstrual pelvic pain) and infertility [Zondervan et al., 2018].

Typically, the diagnostic method or test of the invention is conducted in a subject, preferably a woman, with signs and/or symptoms of endometriosis. The ESHRE guideline: endometriosis (2022) recommends that clinicians should consider the diagnosis of endometriosis in individuals presenting with the following cyclical and non-cyclical signs and symptoms: dysmenorrhoea, deep dyspareunia, dysuria, dyschezia, painful rectal bleeding or haematuria, shoulder tip pain, catamenial pneumothorax, cyclical cough/haemoptysis/chest pain, cyclical scar swelling and pain, fatigue and infertility. In some embodiments, the diagnostic method is conducted in a female subject characterized or preselected for having one, two, three or more of these cyclical and non-cyclical signs and symptoms. For instance, the diagnostic method is conducted in a female subject characterized or preselected for having chronic pelvic pain and/or infertility.

In some embodiments, optionally in combination with any of the above, said subject is not pregnant, and/or has not taken a hormonal treatment in the three months prior to blood extraction and/or does not suffer from any other inflammatory disease, such as inflammatory bowel disease. Said hormonal treatment may for instance be any hormonal treatment typically used for treating endometriosis such as described herein below. These include, but are not limited to, combined (estrogen and progestin) contraceptives, such as combined oral contraceptives (COCs), Gonadotropin-releasing hormone (GnRH) agonists, low doses progesterone (e.g., progestin depot/implant or intrauterine device (IUD)), high doses progesterone, such as in progestin only pills (POPs), or any combination thereof. In preferred embodiments, said subject is not pregnant and does not suffer from any other inflammatory disease.

In some embodiments, endometriosis phenotypes are defined according to the location of the endometriosis type lesions. These include superficial, peritoneal lesions (SUP), ovarian endometriomas (OMA) and deep infiltrating endometriosis (DIE). In preferred embodiments, the method of the invention as described herein is for the screening, diagnosis or monitoring of one or more of superficial, peritoneal lesions (SUP), ovarian endometriomas (OMA) and deep infiltrating endometriosis (DIE), preferably OMA and/or DIE.

In some embodiments, the methods described herein may enable discriminating and classifying subjects according to the predicted presence of endometriosis or endometriosis-like lesions as defined herein. In other embodiments, the methods described herein may enable additionally or alternatively discriminating and classifying subjects according to their distinct degree of endometriosis or endometriosis phenotype.

In some embodiments, the methods described herein may additionally or alternatively enable discriminating and classifying subjects according to disease stage into (i) low-stage disease, which corresponds to stages 1 and 2 according to the revised American Society for Reproductive Medicine (rASRM) classification (Lee SY et al. 2020) and (ii) high-stage disease, which corresponds to stage 3 and 4 according to the revised American Society for Reproductive Medicine (rASRM) classification (Lee SY et al. 2020). Typically, endometrioma, with or without deep-infiltrating endometriosis (DIE), is associated with a high-stage diagnosis whereas the presence of only superficial lesions correlates with low-stage disease:

The subjects identified as having endometriosis, including low-stage or high-stage endometriosis based on the methods described herein can be candidates for further exploratory tests, such as a histologic confirmation after laparoscopy which is the current gold standard (The ESHRE guideline: endometriosis, 2022).

Accordingly, in some embodiments, a method for the screening, diagnosis, or monitoring of endometriosis or endometriosis-like lesions, of predicting the presence of endometriosis type lesions, of predicting the degree of endometriosis or for classifying a subject according to the degree or phenotype of endometriosis as described herein, further comprises conducting an exploratory test, such as a histologic confirmation after laparoscopy, in those subjects identified by the method of the invention as having endometriosis or endometriosis-like lesions.

Step (a) of the methods of the invention comprises or consists of determining in said biological sample the protein levels of BNDF and/or CA125. In preferred embodiments, the biomarkers in step a) consist of CA125 and BDNF.

Brain-derived neurotrophic factor, referred to herein as BDNF, is a secreted dimeric growth factor present in most human tissues, that supports growth and survival of neurons. Similar to other neurotrophins, BDNF is first synthesized as a precursor protein, named pro-BDNF of 32KDa, which is cleaved by different proteases to produce either the mature form of 14KDa, or the truncated form of 28KDa. As used herein, BDNF encompasses mammalian BDNF, including human and functionally equivalent variants thereof such as non-human BDNF, and isoforms or other variants of human and non-human BDNF, including pro-BDNF and mature BDNF (mBDNF), also referred herein as "total BDNF". Functionally equivalent BDNF variants are variants that incorporate alterations, such as, but not limited to, amino acid deletions, additions or substitutions, which do not significantly adversely affect BDNF activity. Amino acid sequences for BDNF are known and readily accessible at sequence databases. Post-translationally modified BNDF is referred to as mature BDNF or mBDNF.

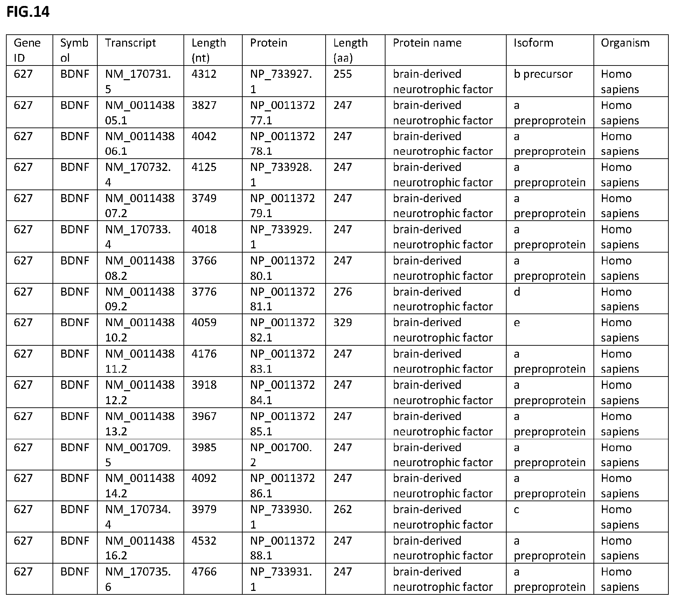

The human BDNF gene (Gene ID: 627) is located in chromosome 11 (11p14.1; Reference GRCh38.p14 Primary Assembly) and has 12 exons. Alternative splicing results in multiple transcript variants; currently, 17 transcript variants have been described as depicted in FIG. 14:

The canonical sequence of the protein expression product of human BDNF gene corresponds to NP_001137277.1 in the NCBI database (See also UniProt P23560-1 entry), a 247 amino acids preproprotein and is herein referred as SEQ ID NO:1:

MTILFLTMVISYFGCMKA**APMKEANIRGQGGLAYPGVRTHGTLESVNGPKAGSRGLTSLA****DTFEHVIEELLDEDQKVRPNEENNKDADLYTSRVMLSSQVPLEPPLLFLLEEYKNYLDAAN****MSMRVRR***HSDPARRGELSVCDSISEWVTAADKKTAVDMSGGTVTVLEKVPVSKGQLKQY**FYETKCNPMGYTKEGCRGIDKRHWNSQCRTTQSYVRALTMDSKKRIGWRFIRIDTSCVCT**LTIKRGR*

Pro-BDNF: in bold; Propeptide: underlined; mBDNF: *in italics*

Precursor form (proBDNF), also referred as SEQ ID NO: 2:

APMKEANIRGQGGLAYPGVRTHGTLESVNGPKAGSRGLTSLADTFEHVIEELLDEDQKVRP

NEENNKDADLYTSRVMLSSQVPLEPPLLFLLEEYKNYLDAANMSMRVRR*HSDPARRGELS*

*VCDSISEWVTAADKKTAVDMSGGTVTVLEKVPVSKGQLKQYFYETKCNPMGYTKEGCRGI*

*DKRHWNSQCRTTQSYVRALTMDSKKRIGWRFIRIDTSCVCTLTIKRGR*

Mature BDNF is produced by proteolytic removal of the propeptide, catalyzed by a FURIN family member.

Sequence of the propeptide (amino acids 19-128), also referred as SEQ ID NO:3

APMKEANIRGQGGLAYPGVRTHGTLESVNGPKAGSRGLTSLADTFEHVIEELLDEDQKVRP

NEENNKDADLYTSRVMLSSQVPLEPPLLFLLEEYKNYLDAANMSMRVRR

Sequence of the mature form (mBDNF), aminoacids 129-247, also referred as SEQ ID NO: 4:

HSDPARRGELSVCDSISEWVTAADKKTAVDMSGGTVTVLEKVPVSKGQLKQYFYETKCNP

MGYTKEGCRGIDKRHWNSQCRTTQSYVRALTMDSKKRIGWRFIRIDTSCVCTLTIKRGR

In some embodiments, determination of BDNF comprises quantifying pro-BDNF and mBDNF, also referred herein as "total BDNF". In some embodiments, the antibodies for total BDNF quantification are obtained by animal immunization using mBDNF as vaccinating antigen. In other embodiments, determination of BDNF comprises quantifying selectively the mature form (mBDNF). In some embodiments, determination of BDNF is conducted using at least one anti-BDNF antibody (e.g., the detection antibody in an ELISA sandwich) which has a higher specificity for the pro-BDNF form than for the mature form. In other embodiments, determination of BDNF is conducted using at least one anti-BDNF antibody (e.g., the detection antibody in an ELISA sandwich) which has a higher specificity for the mature form than for the pro-BDNF form.

Many ELISA kits for the determination of BDNF are known in the art, such as those reflected in Table 1 below (Polachini et al., 2015):

| | R&D System QuantiKine® | Promega BDNF Emax® Immuno-Assay System | Millipore Milliplex® | Millipore ChemiKine™ | Biosensis BDNF Rapid™ ELISA | Aviscera-Bioscience Human BDNF ELISA |
|---|---|---|---|---|---|---|
| Principle of the assay | Sandwich ELISA | Sandwich ELISA | Luminex®/xMAP® technology | Sandwich ELISA | Sandwich ELISA | Sandwich ELISA |
| Sensitivity (pg/ml) | 20 | 15.6 | 2.5 | 7.8 | 2 | 5-8 |
| Range of detection (pg/ml) | 62.5-4000 | 7.8-500 | 12-50000 | 7.8-500 | 7.8-500 | 23-1500 |
| BDNF standard | Human recombinant | BDNF standard (type not declared) | Mix of BDNF 12500 pg + Prolactin | Human recombinant | Human recombinant | Human recombinant |
| Coating/capture Antibody | Pre-coated α-BDNF antibody (mouse monoclonal) | Manually coating with α-BDNF Ab (mouse monoclonal) | Mix of: magnetic beads coated with α-BDNF Ab OR α-Prolactin (type not declared) | Pre-coated α-BDNF antibody (mouse monoclonal) | Pre-coated α-BDNF antibody (mouse monoclonal) | Pre-coated α-BDNF antibody (not type declared) |
| Primary detection antibody | α-BDNF antibody (mouse monoclonal)-HRP conjugated | α-BDNF Ab (chicken polyclonal) | Mix of: Biotinilated α-BDNF antibody AND α-Prolactin (type not declared) | Biotinilated α-BDNF antibody (mouse monoclonal) | Biotinilated α-BDNF antibody (mouse monoclonal) | Biotinilated α-BDNF antibody (type not declared) |
| Type of secondary detection | / | Antibody α-IgY-HRP conjugated | Streptavidin-Phycoerythrin conjugate | Streptavidin-HRP conjugate | Streptavidin-HRP conjugate | Streptavidin-HRP conjugate |
| Sample dilution suggested | 1:20 at least | 1:4 – to user optimization | 1:10 | 1:2 – to user optimization | 1:50 - 1:200 | 1:40 - 1:80 |
| Declared species cross-reactivity | Only human | Not specified | Only human | Human and rat | Human, mouse, rat and others | Only human |
| Processing time | 4-5 hours | 23-24 hours | 19-20 hours | 21-22 hours | 3-4 hours | 6-7 hours |

Table 1. BDNF ELISA Kits. Description and main characteristics of the tested BDNF ELISA kits, as declared by the manufacturers (Polachini et al., 2015).

Polachini et al., 2015 conducted a dot-blot assay and found out that Biosensis, Millipore (both kits) and Promega-Emax® antibodies also reacted with purified pro-BDNF while those from Aviscera-Bioscience and R&D System-Quantikine® showed only marginal reactivity to pro-BDNF. It further discloses that the reactivity of Promega-Emax® (pAB), Biosensis and Sigma against the same amount of pro-BDNF and mature-BDNF (100pg) spotted on the substrate, highlighting a stronger reactivity of these antibodies against pro-BDNF with respect to mature-BDNF.

Cancer antigen 125 (CA125), also referred as MUC16, is a member of the mucin family and a widely used tumour marker in ovarian cancer. Serologic testing for CA-125 has been widely used for detection of endometriosis and monitoring of progressive disease (Barbieri et al., 1986; Kitawaki et al. 2005). It is a membrane-tethered mucin that contains an extracellular domain at its amino terminus, a large tandem repeat domain, and a transmembrane domain with a short cytoplasmic domain. The amino terminus is highly glycosylated, while the repeat region contains 156 amino acid repeats unit that are rich in serines, threonines, and prolines. As used herein, CA125 encompasses mammalian CA125, including human and functionally equivalent variants thereof such as non-human CA125, and isoforms or other variants of human and non-human CA125. Functionally equivalent CA125 variants are variants that incorporate alterations, such as, but not limited to, amino acid deletions, additions or substitutions, which do not significantly adversely affect CA125 activity. Amino acid sequences for CA125 are known and readily accessible at sequence databases.

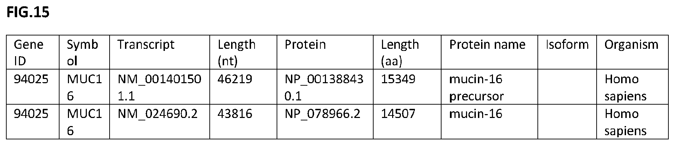

The human CA125 gene (Gene ID: 94025) is located in chromosome 19 (19p13.2; Reference GRCh38.p14 Primary Assembly) and has 90 exons. Alternative splicing has been described to result in 2 transcript variants, namely mucin-16 precursor and mucin-16, as depicted in FIG. 15:

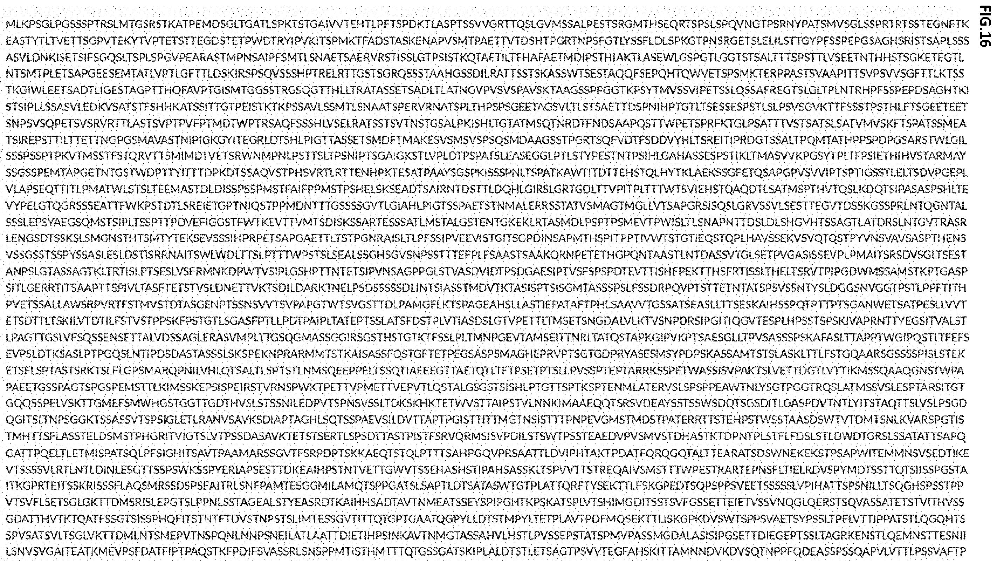

The canonical sequence of the protein expression product of human CA125 gene corresponds to human mucin 16 (GenBank: NP_078966.2, 09-OCT-2022; UniProtKB/Swiss-Prot: accession no. Q8WXI7), which is referred herein as SEQ ID NO: 5 and is depicted in FIG. 16.

Many ELISA kits for the determination of CA125 are known in the art, such as those reflected in Table 2 below:

| | RayBiotech | LifeSpan BioSciences | R&D Systems | R&D Systems | Thermo Fisher Scientific |
|---|---|---|---|---|---|
| Company | | | | | |
| Item | Human CA-125 ELISA | CA125 ELISA Kit | CA125/MU C16 ELISA | CA125/MUC16 ELISA | MUCIN 16/CA125 Human ELISA Kit |
| Catalog Number | ELH-CA125-1 | LS-F24300 | DCA125 | DY5609-05 | EHMUC16 |
| Sensitivity | 0.6 U/ml | 0.938 U/ml | 0.099 U/mL | NA | InterAssay Sensitivity: <12%; IntraAssay Sensitivity: <10% |
| Detection Range | 0.6 U/ml - 400 U/ml | 1.563 - 100 U/ml | 0.50 - 32 U/mL | 31.20 - 2000 pg/mL | 0.55-400 U/mL |
| Quantity | 1 x 96-Well Strip Microplate Kit | 1 plate | 1 Kit | 1 Kit (for 5 Plates) | 96 Tests |
| Reference (supplier webpage) | https://www.raybiotech.com/human-ca-125-elisal?variation n_id=16548 | https://www.lsbio.com/elis akits/human-ca125-sandwich-elisa-elisa-kit-ls-f24300/24300 ?trid=247 | https://www w.rndsyste ms.com/pr oduct-s/human-ca125-muc16-quantikine-elisa-kit dca125 ?utm_sour ce=biocom pare&utm_ medium=re ferral&utm _campaign =product_ DCA125&u tm_term=el isas&utm_ content=ed itorial | https://www.rndsy stems.com/produ cts/human-ca125-muc16-duoset-elisa_dy5609-05?utm_source=b iocompare&utm_ medium=referral& utm_campaign=pr oduct_DY5609-05&utm_term=elis as&utm_content= editorial | https://www.thermofi sher.com/elisa/produ ct/MUCIN-16-CA125-Human-ELISA-Kit/EHMUC16 |

Preferably, the protein levels of CA125 and BNDF are determined in a serum sample, more preferably in the same serum sample. In some embodiments said serum sample is a venous serum sample. Typically, that serum is obtained from blood samples collected after fasting, for instance after fasting for at least 10 hours.

These types of samples are routinely used in the clinical practice and a person skilled in the art will know how to identify the most appropriate means for their obtaining and preservation. Once a sample has been obtained, it may be used fresh, it may be frozen or preserved using appropriate means.

The biological sample, preferably a serum sample, can be a sample obtained from a subject at any time of the menstrual cycle, including the menstrual, proliferative or secretory phase (e.g., early secretory, mid secretory or late secretory phase), Mihm M, Gangooly S, Muttukrishna S. The normal menstrual cycle in women. Anim Reprod Sci. 2011 Apr;124(3-4):229-36. In some embodiments, the sample is from the proliferative or secretory phase, preferably from the proliferative or mid secretory phase.

Suitable methods for determining the levels of a given protein include, without limitation, those described herein below. Preferred methods for determining the protein expression levels in the methods of the present invention are immunoassays. Various types of immunoassays are known to one skilled in the art for the quantitation of proteins of interest. These methods are based on the use of affinity reagents, which may be any antibody or ligand specifically binding to the target protein or to a fragment thereof, wherein said affinity reagent is preferably labeled. Illustrative, but non-exclusive, examples of labels that can be used include radioactive isotopes, enzymes, fluorophores, chemoluminescent reagents, enzyme cofactors or substrates, enzyme inhibitors, particles, dyes, etc.

Affinity reagents may be any antibody or ligand specifically binding to the target protein or to a fragment thereof. Affinity ligands may include proteins, peptides, peptide aptamers, affimers and other target specific protein scaffolds, like antibody-mimetics.

Specific antibodies against the protein markers used in the methods of the invention may be produced for example by immunizing a host with a protein of the present invention or a fragment thereof. Likewise, peptides specific against the protein markers used in the methods of the invention may be produced by screening synthetic peptide libraries.

Western blot or immunoblotting techniques allow comparison of relative abundance of proteins separated by an electrophoretic gel (e.g., native proteins by 3-D structure or denatured proteins by the length of the polypeptide). Immunoblotting techniques use antibodies (or other specific ligands in related techniques) to identify target proteins among a number of unrelated protein species. They involve identification of protein target via antigen-antibody (or protein-ligand) specific reactions. Proteins are typically separated by electrophoresis and transferred onto a sheet of polymeric material (generally nitrocellulose, nylon, or polyvinylidene difluoride). Dot and slot blots are simplified procedures in which protein samples are not separated by electrophoresis but immobilized directly onto a membrane.

Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay. Multiplex and any next generation versions of any of the above, such as bead-based flow-cytometry immunoassays (e.g., based on the Luminex xMAP technology) are specifically encompassed. In a particular embodiment, said immunoassay is an ELISA assay or any multiplex version thereof.

Other methods that can be used for quantification of proteins in solution are techniques based on mass spectrometry (MS) such as liquid chromatography coupled to mass spectrometry (LC / MS), described for example in US2010/0173786, or tandem LC-MS / MS (WO2012/155019, US2011/0039287, M. Rauh, J Chromatogr B Analyt Technol Biomed Life Sci 2012 February 1, 883-884. 59-67) and multiplex versions of the above techniques, as well as the next generation of such techniques and combinations thereof.

In other embodiments, determination of whether CA125 and/or BDNF protein levels are increased with respect to a reference value is conducted by lateral flow tests, also known as Lateral Flow Immunochromatography (LFIC) tests or just Lateral Flow Immunoassays (LFI). These are simple small devices intended to detect a target analyte in a given sample. These tests are simple, fast, and cheap and do not require specialized and costly equipment nor qualified personnel. In the beginning, LFIC tests were exclusively used in medical diagnostics (either for home testing, point of care testing, or laboratory use) but their presence in other areas are now very common. The first application was the well-known home pregnancy test. Although there are several commercially available semi-quantitative tests, LFIC tests are mainly qualitative tests and they just give a positive or negative result based on the presence or absence of the target analyte at a specific concentration.

The technology is based on a series of capillary beds with porous materials that has the capacity to transport liquids spontaneously. When the target analyte is big (e.g., a protein) a sandwich format is commonly used. Usually, the first element (the conjugate pad) has stored the so-called colloids, a dried format of bioactive particles (mainly gold nanoparticles or latex microparticles bound to a specific antibody against the target analyte) in a salt-sugar matrix. This conjugate pad usually acts also as the sample pad and it contains everything to facilitate the recognition of the target antigen by the colloid. Once the conjugate pad is soaked by the sample, the fluid dissolves the salt-sugar matrix and the colloids. Therefore, the sample and the colloid mix while they flow through the porous structure. In this way, the analyte binds to the colloid and the complex is transported into the nitrocellulose membrane. This material has one specific area called test line where a third molecule (usually another different antibody against the target analyte) has been immobilized. When the complex (colloid-antigen) reaches the test line it gets bound to this antibody and after a while, when more and more fluid has flown, colloids accumulate, and the test line acquires color. Typically, there is also a control line (after the test line) that captures the colloids that have not bound to the test line. In the control line, a fourth molecule (could be an antibody against the Fc fraction of the first antibody) is adsorbed and its color acquisition ensures the test functionality. After passing these reaction zones the fluid enters the final porous material, the wick or sink that simply acts as a waste container.

The expression "determining the protein levels" or "determining the protein expression levels" as used herein, refers to ascertaining the absolute or relative amount or concentration of the protein biomarker in the sample. In some embodiments, the protein levels are expressed in logarithmic form. Techniques to assay levels of individual biomarkers from test samples are well known to the skilled technician, and the invention is not limited by the means by which the components are assessed.

Expression levels may be absolute or relative. When the expression levels are normalized, normalization can be performed with respect to different measures in the sample. These procedures are well known to one skilled in the art. Typically, expression levels are normalized with respect to an "endogenous control". An "endogenous control" as used herein may relate to a gene expression product whose expression levels do not change or change only in limited amounts in endometriosis subjects with respect to healthy subjects.

"Endogenous control", also referred herein as "control gene" or "normalizing gene" is usually the expression product from a housekeeping gene and which codes for a protein which is constitutively expressed and carries out essential cellular functions. Housekeeping genes that can be used as endogenous control include for example β-2-microglobulin, ubiquitin, 18-S ribosomal protein, cyclophilin, GAPDH, actin and HPRT. In a particular embodiment, "endogenous controls" used in the present invention are selected from the group consisting of 18S, ACTB, B2M, GAPDH, GUSB2, HPRT, PPIA, RPLP, TBP, TUBB, UBC, and YWHAZ. In other instances, normalization can be conducted by total protein normalization.

In a particular embodiment, BDNF and/or CA125 determination is conducted in human serum by a sandwich ELISA immunoassay. In some embodiments, each biomarker is determined in a different set of wells. That is, the ELISA-plate is coated with an antibody directed against either BDNF or CA125. In other embodiments, wells have a double coating, namely, each well is coated with an antibody directed against BDNF and another antibody against CA125.

In some embodiments, BDNF or CA125 from samples, and the standards when used, bind to the antibodies and are immobilized on the plate. After removal of the unbound proteins in the biological sample (e.g., serum) by a washing procedure, the plates are incubated with biotin conjugated anti-human CA125 and biotin conjugated anti-human BDNF antibodies. Unbound biotin conjugate is washed off with washing solution. In a further step, streptavidin-HRP conjugate is added, and binds to biotin. Unbound streptavidin-HRP is washed off with washing solution. Finally, a substrate solution is added, and the formed complex catalyzes the chemical reaction of the substrate into a colored chemical entity. The enzymatic color reaction is stopped after a defined period of time. The concentration of the colored chemical correlating proportionally to the concentration of the antibody is measured photometrically.

The methods of the invention may optionally comprise **step (b)** which comprises calculating a score. The score is a value obtained according to a given mathematical algorithm wherein the protein values of each of the biomarkers used in the methods of the invention are variables of said mathematical algorithm. Preferably, the formula for calculating said score comprises calculating the sum of the product between the biomarker protein levels and their estimated regression coefficients obtained in a regression analysis.

Furthermore, **step (c)** of the methods of the invention comprises classifying/selecting the subject as having, or suspected to have, endometriosis or endometriosis-like lesions based on the CA125 and BDNF protein levels in the subject's sample as determined in step a), or on the score obtained in b).

Typically, in step c) said method comprises comparing the CA125 and BDNF protein levels in a), or the score in b), of the subject sample with a reference value; and an increase of the CA125 and BDNF protein levels, or the score, in the subject sample with regard to said reference value is indicative of endometriosis or endometriosis-like lesions; whereas a decrease of the CA125 and BDNF protein levels, or the score, in the subject sample with regard to said reference value is indicative of absence of endometriosis or endometriosis-like lesions.

Accordingly, the methods described herein comprise comparing the protein levels or score in the subject sample with a reference value. The term "reference value", as used herein, relates to a predetermined criterium used as a reference for evaluating the values or data obtained from the samples collected from a subject. This "reference value" may also be referred as "cut-off value" or "threshold value".

The term "reference value" may refer to a value determined in one or more samples, generally mean values of preferably a statistically significant number of samples, obtained from a reference or control population. In some embodiments, the "reference or control population" is a group of individuals who have symptoms of endometriosis, but are negative to a laparoscopy with histologic confirmation.

Determination of the "reference value" is carried out under similar or identical conditions as the biological sample of the test subject. Typically, said reference samples are from the same type of biological sample, e.g., the same tissue or cell type and have undergone the same procedures for their obtaining and preservation. Alternatively, said "reference values" are pre-established values, generally mean values, in the control or reference population.

The reference value can be an absolute value, a relative value, a value that has an upper or a lower limit, a range of values, an average value, a median value, a mean value, a z-score (e.g. mean value + or - 1, 2 or 3 standard deviation (SD)), a tertile value, or a value as compared to a particular control or baseline value.

In addition, it is further noted that a variety of statistical and mathematical methods for establishing the threshold or cut-off level of expression are known in the prior art. In some embodiments, a threshold or cut-off expression level for a particular biomarker may be selected based on data from Receiver Operating Characteristic (ROC) plots, for instance by determining the optimal cut-off value, as determined at a maximum Youden's index. One of skill in the art will appreciate that these threshold or cut-off expression levels can be varied, for example, by moving along the ROC plot for a particular biomarker or combinations thereof, to obtain different values for sensitivity or specificity thereby affecting overall assay performance.

Sensitivity, specificity, and/or accuracy are parameters typically used to describe the validity or performance of a test. In particular, they are used to quantify how good and reliable the discrimination method is. A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

In preferred embodiments, the methods of the invention have sensitivity, specificity and/or accuracy values of at least about 60 %, preferably of at least about 70 %, and can be, for example, at least 75 %, at least 80 %, at least 85 %, at least 90 %, at least 95 %, at least 96 %, at least 97 %, at least 98 %, at least 99 % or 100% in at least 60 % of the group or population assayed, or preferably in at least 65 %, 70 %, 75 %, 80 %, 85 %, 90 %, 95 % or 100 % of the group or population assayed.

In the methods of the invention, the protein levels or the score are considered "decreased" when said value is lower than a reference value. Preferably, the value is considered to be lower than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference value.

Likewise, in the context of the methods of the invention, the protein levels or the score is considered "increased" when said value is higher than a reference value. Preferably, the value is considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Alternatively, or in addition, subjects having more than about 1.1, 1.2, 1.3, 1.4, 1.5, 2, 3, 4, 5, 6, 7, 8, 9, 10, 15 or 20 fold levels deviation (i.e., increase or decrease) than the reference value as described herein.

The method of the invention, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical significance measures obtained by statistical tests; illustrative, non-limiting examples of said statistical significance measures include determining confidence intervals, determining the p-value, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

It is further noted that the accuracy of the method of the invention can be further increased by additionally considering other gene or protein markers, biochemical parameters and/or variables related to the subject's history and/or clinical characteristics (e.g., age, sex, tobacco use and/or other risk factors), preferably wherein said gene or protein biomarkers are other than ZAG and/or glycodelyn. Determination of these other markers, parameters and/or characteristics can be sequential or simultaneous to any or all of the steps described herein above.

In preferred embodiments, the method of the invention further comprises determining one or more variables related to the subject's history and/or clinical characteristics, preferably, these variables are one, two, three, four, five, six, seven or eight selected from the group consisting of:

- Record of previous surgery for endometriosis;

- Painful periods as a symptom for endometriosis examination;
- Hormonal implant use;
- Frequency of harder stools in case of period pain in the last 3 months;
- Age at first experience of intercourse pain;
- Age at first experience of ovarian cyst symptoms;
- Severity of menstrual pain last time (1-10); andStarting age of ibuprofen use.

As shown in Example 3, the response to these variables has been shown to be associated to presence of endometriosis. In particular,

a) record of previous surgery for endometriosis; possible answer: Yes/No. Yes: associated with endometriosis. No: not associated with endometriosis.

b) painful periods as a symptom for endometriosis examination; possible answer:
Yes/No. Yes: associated with endometriosis. No: not associated with endometriosis.

c) hormonal implant use (refers to estrogen and/or progestin hormonal implant use); possible answer: Yes/No. Yes: not associated with endometriosis. No: associated with endometriosis.

d) frequency of harder stools in case of period pain in the last 3 months; possible answer: Common (sometimes, often and most of the time answers were grouped in this class)/Not common (never/rarely answers were grouped in this class). Common: associated with endometriosis. Not common: not associated with endometriosis.

e) age at first experience of intercourse pain; at higher age, higher association with endometriosis

f) age at first experience of ovarian cyst symptoms; at higher age, higher association with endometriosis

g) severity of menstrual pain last time (1-10); possible answer: none, mild, moderated or severe. None and mild (Pain 0-5): not related with endometriosis. Moderate and severe (Pain 6 to 10): related with endometriosis.

h) starting age of ibuprofen use; at higher age, higher association with endometriosis.

In some embodiments, said variables are one, two, three or four selected from the group consisting of:

a) record of previous surgery for endometriosis;
b) painful periods as a symptom for endometriosis examination;
c) Severity of menstrual pain last time (1-10); and
d) Starting age of ibuprofen use.

In other embodiments, said variables are one, two, three or four selected from the group consisting of:

a) record of previous surgery for endometriosis;
b) painful periods as a symptom for endometriosis examination;
c) Hormonal implant use; and
d) Frequency of harder stools in case of period pain in the last 3 months.

For instance, said variables may consist of:

- record of previous surgery for endometriosis; and
- painful periods as a symptom for endometriosis examination; or
- record of previous surgery for endometriosis; and
- hormonal implant use; or
- record of previous surgery for endometriosis; and
- frequency of harder stools in case of period pain in the last 3 months; or
- painful periods as a symptom for endometriosis examination; and
- hormonal implant use; or
- painful periods as a symptom for endometriosis examination; and
- frequency of harder stools in case of period pain in the last 3 months; or

- hormonal implant use; and
- frequency of harder stools in case of period pain in the last 3 months; or
- record of previous surgery for endometriosis;
- painful periods as a symptom for endometriosis examination; and
- hormonal implant use; or
- record of previous surgery for endometriosis;
- painful periods as a symptom for endometriosis examination; and
- frequency of harder stools in case of period pain in the last 3 months; or
- record of previous surgery for endometriosis;
- hormonal implant use; and
- frequency of harder stools in case of period pain in the last 3 months; or
- painful periods as a symptom for endometriosis examination;
- hormonal implant use; and
- frequency of harder stools in case of period pain in the last 3 months.

The subject in the methods of the invention is preferably a female subject, more preferably a human female subject presenting signs and/or symptoms of endometriosis as described herein above.

The methods of the present invention or any of the steps thereof might be implemented by a computer. In a particular embodiment, optionally in combination with any of the features or embodiments as described herein, the score obtained by mathematical combination of the determined protein levels, and optionally in combination with other gene or protein markers, biochemical parameters, and/or variables related to the subject's history and/or clinical characteristics, preferably wherein said gene or protein biomarkers are other than ZAG and/or glycodelyn, is calculated using a computer; and/or the comparison between the values in the subject sample and the reference value is conducted using a computer.

Therefore, a further aspect of the invention refers to a computer implemented method, wherein the method is any of the methods disclosed herein or any combination thereof.

It is noted that any computer program capable of implementing any of the methods of the present invention or used to implement any of these methods or any combination thereof, also forms part of the present invention.

This computer program may be web-based or directly loadable into the internal memory of a digital computer, comprising software code portions for performing the steps of comparing the values from the one or more biological samples of a subject with a reference value and determining endometriosis or presence of endometriosis-like lesions, or the degree or stage of endometriosis disease, as described herein above, when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. The present invention further relates to a computer-readable storage medium having stored thereon a computer program of the invention or the results of any of the methods of the invention.

As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

In some embodiments, the present invention refers to a computer implemented (CI) method of the invention as described herein above, wherein said method comprises:

i. receiving by a computer program the information on the protein levels of a group of biomarkers in a biological sample (preferably a serum sample) isolated from the subject, wherein said biomarkers consist of BDNF and CA125, and optionally information on other gene or protein markers, biochemical parameters and/or variables related to the subject's history and/or clinical characteristics;

ii. processing the information received according to step i) by conducting step c), and optionally step b), as described herein above;

iii. generate an output information, for instance through a terminal display,

In preferred embodiments, said method is a method for the screening, diagnosis or monitoring of endometriosis or endometriosis type lesions as described herein and the generated output is identifying or classifying a subject having or with a high likelihood to have endometriosis or as an individual who could benefit from an exploratory test, such as a histologic confirmation after laparoscopy.

In preferred embodiments it also refers to a computer program comprising instructions which, when the program is executed by a computer, cause the computer to carry out steps ii) and/or iii), of the _CII method described herein above.

In some embodiments also refers to a data-processing apparatus comprising means for carrying out steps ii) and/or iii) of the CI invention described herein above.

*Methods of subject selection and treatment of the invention*

In a further aspect, the present invention also relates to a method for selecting a subject which would likely benefit from a treatment suitable for treating endometriosis or endometriosis-like lesions, wherein said subject is selected as having or suspected to have endometriosis or endometriosis-like lesions according to a method as described herein above.

In a related aspect, the invention also pertains to a method for treating a subject with or suspected to have endometriosis or endometriosis-like lesions by administering a therapeutically effective amount of a suitable treatment or by conducting a surgical resection, wherein said subject is a subject which has been classified as having or suspected to have endometriosis or endometriosis-like lesions by a method as described herein above.

Among several interventions, suitable treatments for treating endometriosis or endometriosis-like lesions include combined (estrogen and progestin) contraceptives (first-line), progestin-only therapy, Gonadotropin-releasing hormone (GnRH) agonists, Gonadotropin-releasing hormone antagonists, danazol, Aromatase inhibitors and Neuropathic pain treatments. The estrogen may comprise ethinyl estradiol, mestranol, quinestranol, estradiol, estrone, estran, estriol, estetrol, conjugated equine estrogens and precursors thereof that are capable of releasing such an estrogen in vivo. The progestogen may comprise levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagestone, chlormadinone, cyproterone, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone, megestrol, melengestrol, nomegestrol, norethindrone (=norethisterone), norethynodrel, norgestrel (includes d-norgestrel and dl-norgestrel), norgestrienone, normethisterone, progesterone, quingestanol, (17alpha)-17-hydroxy-11-methylene-19-norpregna-4,15-diene-20-yn-3-one, tibolone, trimegestone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime and precursors of these compounds. Gonadotropin-releasing hormone (GnRH) agonists may comprise buserelin, leuprorelin, nafarelin, and triptorelin. Gonadotropin-releasing hormone antagonists may comprise cetrorelix, elagolix, ganirelix, linzagolix and relugolix. Aromatase inhibitors may comprise aminoglutethimide, anastrozole, exemestane, vorozole, letrozole, fadrozole, rogletimide, atamestane, formestane and liarozole.

Apart from providing a histologic diagnosis, surgical resection reduces pain by destroying the endometriotic implants. Surgical resection can be conservative (treatment of endometriosis lesions by ablation or resection), definitive (hysterectomy, with or without oophorectomy, in addition to resection of endometriosis), or radical (removal of all visible implants at time of surgery).

In a further aspect, the present invention also relates to a method for selecting a subject which would likely benefit from a confirmation test, wherein said subject is a subject which has been classified as having or suspected to have endometriosis or endometriosis-like lesions according to a method as described herein above. Said confirmatory test can be any confirmatory tests known in the art, preferably a histologic confirmation after laparoscopy.

In a related aspect, the invention also relates to a method of conducting a confirmatory test, such as a histologic confirmation after laparoscopy., in a subject suspected of having endometriosis or endometriosis-like lesions, wherein said subject has been selected as having endometriosis or endometriosis-like lesions by a method as described herein above.

*Kits and uses of the invention*

In a further aspect, the present invention refers to the use of kit for determining the levels of one or more of the biomarkers as described herein in a biological sample as described herein (preferably a serum sample) isolated from a subject. Combinations of markers can be as described above under the methods of the invention. The kit may also contain instructions indicating how the materials within the kit may be used.

The term "kit" or "testing kit" denotes combinations of reagents and adjuvants required for an analysis. Although a test kit consists in most cases of several units, one-piece analysis elements are also available, which must likewise be regarded as testing kits.

In a particular embodiment, said kit is suitable for determining the protein levels of CA125 and/or BDNF in a biological sample, preferably a serum sample, as described herein above, and said kit comprises:

    i. a reagent for the quantification of the BDNF protein levels; and/or

ii. a reagent for the quantification of the CA125 protein levels;

iii. optionally, a reagent for the quantification of a protein for normalization purposes;

iv. optionally, a human BDNF and a human CA125 (preferably a recombinant version thereof, such as the WHO Reference Reagent (WHO RR) for brain derived neurotrophic factor (BDNF) or recombinant human BDNF protein (cat no. B-250, Alomone); and/or recombinant human CA125/MUC16 Protein (cat no. 5609, R&D systems), for instance for calibration purposes and/or as positive controls;

v. optionally, further comprising instructions for the use of said reagents in determining the levels of said proteins in a biological sample isolated from a subject.

Particular and preferred embodiments of the kit and the uses thereof are as described herein for the methods of the invention.

In some instances, human BDNF and human CA125 are provided for calibration purposes as a series of calibration solutions with increasing concentration values of the corresponding protein. In some embodiments, the kit of the invention comprises a common calibrator solution in which both BDNF and CA125 are present.

In preferred embodiments, said kit comprises:

a) an affinity reagent for human BDNF, preferably for SEQ ID NO: 2;

b) an affinity reagent for human CA125, preferably for SEQ ID NO: 4;

c) optionally, an affinity reagent for a protein for normalization purposes;

d) optionally, a human BDNF and a human CA125 (preferably a recombinant version thereof), for instance for calibration purposes and/or as positive controls;

e) optionally, further comprising instructions for the use of said reagents in determining the levels of said proteins in a biological sample isolated from a subject.

Said reagents, as well as preferred features and embodiments of the kit and the uses thereof may be as described herein above for the methods of the invention.

The term "an affinity reagent for" as used herein refers to an affinity reagent capable of specifically binding to the recited target protein. The various affinity reagents may be labelled with the same or different tags. Preferably, these will be labelled with different tags for multiplex analysis.

In preferred embodiments, said affinity reagent is a polypeptide, preferably an antibody (e.g. a monoclonal antibody), capable of specifically binding to the recited target proteins. Numerous methods known to those skilled in the art are available for obtaining antibodies or antigen-binding fragments thereof. Antibodies can be produced using recombinant DNA methods, see, e.g., Current Trends in Monoclonal Antibody Development (Shire et al. 2010). Monoclonal antibodies may also be produced by preparing immortalized cell lines capable of producing antibodies having the desired specificity. Such immortalized cell lines may be produced in a variety of ways. Conveniently, a small non-human animal, such as a mouse, is hyperimmunized with the desired immunogen. The vertebrate is then sacrificed, usually several days after the final immunization, the spleen cells removed, and the spleen cells immortalized. The most common technique is fusion with a myeloma cell fusion partner, as first described by (Köhler and Milstein 1975)). Other techniques, including EBV transformation, transformation with bare DNA, e.g., oncogenes, retroviruses, etc., or any other method which provides for stable maintenance of the cell line and production of monoclonal antibodies are also well known. Specific techniques for preparing monoclonal antibodies are described in (Harlow and Lane 1988).

Antibodies specifically binding to each of BDNF and CA125 are known in the art. Non-limiting examples include the commercially available antibodies described herein above. Preferably, said kit comprises a solid support or surface which is coated with an affinity reagent as described herein above (i.e., the primary or capturing affinity reagent). The solid support can include any support known in the art on which said affinity reagent can be immobilized. In some embodiments, said solid supports are microtiter well plates, slides (e.g., glass slides), chips (e.g., protein chips, biosensor chips, such as Biacore chips), microfluidic cartridges, cuvettes, beads (e.g., magnetic beads, xMAP® beads) or resins.

Said reagent for determining the levels of the marker protein as defined herein above may also be a secondary (detection) affinity reagent or antibody. These antibodies can be monoclonal or polyclonal antibodies. Also, these can be derived from any mammalian organism, including mice, rats, hamsters, goats, camels, chicken, rabbit, and others. Typically, the primary antibody and the secondary antibody will be from different animal species. These may also be monoclonal antibodies, with the proviso that, these bind to different epitopes and there is no steric interference. Secondary or detection antibodies can be conjugated to any suitable detection means such as enzymes (e.g., horseradish peroxidase (HRP), alkaline phosphatase (AP), luciferase, and the like) or dyes (e.g., colorimetric dyes, fluorescent dyes, fluorescence resonance energy transfer (FRET)-dyes, time-resolved (TR)-FRET dyes, and the like). Other suitable labels have been described herein above.

In some embodiments, said kit is an ELISA kit which comprises:

i.an anti-BDNF capture antibody and an anti-CA125 capture antibody;

ii.an anti-BDNF detection antibody and an anti-CA125 detection antibody, wherein the detection antibody is conjugated to a detection tag, such as an enzyme (e.g., peroxidase or alkaline phosphatase) or biotin;

iii. an enzyme substrate, such as tetramethylbenzidine (TMBD);

iv. when the detection antibody is conjugated to biotin, an enzyme conjugated streptavidin;

v. optionally, a recombinant human BDNF and a recombinant human CA125 for the calibrator and the positive controls;

vi. optionally, further comprising instructions for the use of said reagents in determining the protein levels of BDNF and CA125 in a biological sample isolated from a subject as described herein.

In some embodiments, said kit for determining the levels of a target protein of the invention in a biological sample, is a lateral flow immunoassay (LFI) which may optionally be coupled to a line intensity reader, and enables determining the levels of the target protein or when levels of the target protein are above a reference level. The technology as well as typical reagents are as described herein above. To practice the present invention, a large variety of different lateral flow system devices known in the art could be used.

Ancillary reagents typically used in an immunoassay, such as a solid support immunoassay, may also be comprised and include, e.g., an immobilization buffer, an immobilization reagent, a dilution buffer, a secondary or detection antibody, a detection reagent, a blocking buffer, a washing buffer, a detection buffer, a stop solution, a system rinse buffer, and a system cleaning solution which are well known by a person skilled in the art.

In still a further aspect, the invention relates to the use of a kit as described above in any of the methods as described herein, such as a method for the screening, diagnosing or monitoring of endometriosis or endometriosis-like lesions in a subject; a method for predicting the presence of endometriosis type lesions, a method of predicting the degree of endometriosis or for classifying a subject according to the degree or phenotype of endometriosis as described herein; a method for subject selection and treatment or a method for subject selection and performing an exploratory test (such as a histologic confirmation after laparoscopy) as described herein above .

It is contemplated that any features described herein can optionally be combined with any of the embodiments of any method, kit, use of a kit, or computer program of the invention; and any embodiment discussed in this specification can be implemented with respect to any of these. It will be understood that particular embodiments described herein are shown by way of illustration and not as limitations of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB.

Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by $\pm 1, 2, 3, 4, 5, 6, 7, 8, 9$, or 10%. Accordingly, the term "about" may mean the indicated value $\pm 5\%$ of its value, preferably the indicated value $\pm 2\%$ of its value, most preferably the term "about" means exactly the indicated value ($\pm 0\%$). The following examples serve to illustrate the present invention and should not be construed as limiting the scope thereof.

## EXAMPLES

### Example 1.- Evaluation of CA125, BDNF, ZAG peptide, glycodelin/PP14 in serum and plasma as biomarkers of endometriosis

In this study, a panel of four biomarkers (CA125 (Cancer antigen 125), BDNF (Brain-derived neurotrophic factor), ZAG (Zinc-$\alpha$-2-glycoprotein) peptide and PP14 (Glycodelin/Placental protein 14) in serum and in plasma was evaluated for its ability to distinguish patients with endometriosis (n = 137) from healthy controls (n = 68), with samples taken from individuals residing in the United Kingdom (primary outcome). In addition, the association between the biomarker concentrations and patient characteristics was examined, a pairwise comparison of biomarker levels in serum and plasma was made.

### Material and methods

Definition of cases and control groups.

This prospective study was conducted on serum samples acquired from the Oxford Endometriosis CaRe Centre biobank (UK). The experimental protocol was approved by ethics committee CElm HM Hospitales.

204 serum samples obtained from patients of reproductive age (18 - 50 years old) with suspicion of endometriosis in which the disease has been confirmed or excluded by histological evaluation of excised lesions during laparoscopy. Patients were pre-classified as controls and cases and anonymized in the biobank: Case/control classification was performed based on laparoscopy and histological verification of endometrial glands and/or stroma in the excised lesions. Patients were classified as cases if the endometriosis was confirmed by laparoscopy and histology, and as controls if endometriosis could not be confirmed by laparoscopy and histology. Patients were excluded if they had taken a hormonal treatment in the three months prior to blood extraction. Analysis was performed blinded to the surgical and imaging findings. The patients with endometriosis were stratified according to the revised American Society of Reproductive Medicine (rASRM) classification. The main characteristics of the patients included in this study are displayed in Table 1.

Table 1. Demographic characteristics of the patients in the Oxford Endometriosis CaRe

|  | Controls<br>N=68 | Endometriosis<br>N=136 |
|---|---|---|
| Age years (mean $\pm$ SD) | 33.5 (5.96) | 35.6 (6.42) |
| BMI (mean $\pm$ SD) | 25.38 (4.63) | 26.46 (5.32) |
| rASRM classification<br>I-II<br>III-IV | -<br>- | 68 (50%)<br>68 (50%) |
| Control diagnosis | | |

Note. BMI= Body Mass Index; rASRM= revised American Society for Reproductive Medicine Centre biobank cohort1.

Sample collection, preservation and storage

The specimens were collected and handled following the World Endometriosis Research Foundation Standard Operating procedures (Rahmioglu et al. 2014) after receiving patients' consent. Patients were asked to fast for at least 10 hours prior to blood collection. Serum samples were stored in a biobank at -80 °C for up to 5 years and were transferred to the laboratory analysis site.

Biomarker concentration determination.

Mean concentration values of the biomarkers in the isolated biological samples from cases and controls were determined using commercial ELISA kits according to the manufacturer's instructions.

**Table 1. ELISA kits used for total BDNF, CA125, Glycodelin and ZAG determination**

| Analyte | Kit | Catalogue number | Supplier |
|---|---|---|---|
| Total BDNF | ChemiKine™ BDNF Sandwich ELISA | CYT306 | EMD (Merck) Millipore, Darmstadt, Germany |
| CA125 | Human CA125 Quantikine® ELISA | DCA125 | R&D Systems, Abingdon, UK |
| Glycodelin | Glycodelin ELISA | HUFI02772 | ELISAGenie (London, United Kingdom) |
| ZAG | Human ZAG ELISA kit | BMS2201 | Thermo Fisher, Bleiswijk, the Netherlands |

Methods of statistical analysis

The non-parametric two-sided Mann-Whitney U test, also known as Wilcoxon rank-sum test, was implemented with the wilcox.test function from the stats package. The null hypothesis is that the distributions of the case predictor values, and the control predictor values differ by a location shift of 0 (no shift), with the alternative that they differ by a location shift different from 0. From the resulting W (or U) statistic, a p value is calculated. The difference in location represents the estimated median of the differences between a case sample and a control sample.

For each numerical (quantitative) variable, univariate logistic regression was performed using the glm function from the stats package on default settings (family = "binomial"). With the resulting model, the probabilities of diagnosis were calculated for each data point with the predict function, and the resulting dataframe was applied on the roc function from the pROC package to obtain sensitivity (TP/(TP + FN)) and specificity (TN/(TN + FP)) values across a range of threshold values. The true positive (TP), true negative (TN), false positive (FP) and false negative (FN) results were obtained, together with Youden's index (sensitivity + specificity - 1), accuracy ((TP + TN)/(TP + TN + FP + FN)), positive (sensitivity/(1 - specificity)) and negative likelihood ratio (specificity/(1-sensitivity)), diagnostic odds ratio ((TP x TN) / (FP x FN)), the positive (TP/(TP + FP)) and negative (TN/(TN + FN)) predictive values, according to the standard definitions. Predictive values were also calculated based on an estimated true prevalence of 24% or 50% for positive diagnosis of endometriosis:

$$tPPV = \frac{tPrev \times Sensitivity}{tPrev \times Sensitivity + (1 - tPrev) \times (1 - Specificity)}$$

$$tNPV = \frac{(1 - tPrev) \times Specificity}{(1 - tPrev) \times Specificity + tPrev \times (1 - Sensitivity)}$$

The receiver-operator characteristic (ROC) curve was plotted based on the true positive rate (sensitivity) and false positive rate (1 - specificity) along the spectrum of threshold values. The area under the ROC curve (AUC) was calculated using the auc function of the pROC package.

Confidence intervals for proportions were obtained with the binom.confint function from the binom package, or from applying the diagnostic function from the ThresholdROC package. For tPPV and tNPV confidence interval estimations, the sample size was set at respectively TP + FP and TN + FN.

Chi-squared analysis was performed using the chisq.test function (stats package) on categorical variables to identify a pairwise statistically significant difference in diagnostic outcome between the categories. In case of ordered categorical data, the Cochran-Armitage test was used, based on function CochranArmitageTest from the DescTools package.

To evaluate for associations between predictors, chi-squared analysis (or Cochran-Armitage testing) was used when comparing two categorical variables, the Mann-Whitney U test when comparing a numerical with a categorical variable, and Pearson's correlation coefficient (cor function from stats package) when comparing two numerical variables.

Because of the large number of (non-independent) pairwise comparisons, the p values were adjusted using the Benjamini-Hochberg correction for analyses involving categorical variables (vs outcome, vs numerical variable, vs other categorical variable), with corrections for p values grouped by categorical parameter.

To assess whether both CA125 and BDNF are important contributors (with statistical significance) in a logistic regression model for endometriosis diagnosis, the glm function from the stats package was applied with log transformed CA125 and BDNF as input variables and endometriosis diagnosis as outcome. Next, the step function from the stats package was used at the default settings to evaluate whether both parameters contributed meaningfully to the model.

The glm function from the stats package was used to generate a logistic regression model without interaction terms with the selected input variables. After training, the models were tested on the isolated testing data using the predict function and subsequently, the roc function was applied to analyze different performance outcomes (AUC, optimal Youden's index, maximum accuracy, sensitivity at 95% specificity, specificity at 95% sensitivity).

The final model was created by including all individuals for development of a logistic regression model. The associated performance metrics values were also reported.

### Results

1.1. Data overview and data availability (n)

**Table 2. -Data overview and data availability (n)**

| General | | | |
|---|---|---|---|
| **Parameter** | **n** | **Parameter** | **n** |
| Stage | 204 | Age at surgery | 204 |
| Date of sampling | 204 | Body-mass index (BMI) | 44 |
| | | | |
| **Biomarkers** | | | |
| **Parameter** | **n** | **Parameter** | **n** |
| Serum CA125 | 203 | Plasma CA125 | 204 |
| Serum Glycodelin | 197 | Plasma Glycodelin | 202 |
| Serum total BDNF | 204 | Plasma total BDNF | 204 |
| Serum ZAG | 204 | Plasma ZAG | 204 |
| | | | |
| **Hormone treatment** | | | |
| **Parameter** | **n** | **Parameter** | **n** |
| Hormonal treatment (ever) | 176 | Type: progestin injection | 205 |
| Starting age of treatment | 147 | Type: danazol | 205 |
| Cumulative years of treatment | 118 | Type: birth control pill (type unknown) | 205 |
| Reason: contraception | 187 | Type: progestin-only pill | 205 |
| Reason: chronic pelvic pain | 187 | Type: GnRH agonist | 205 |
| Reason: irregular cycles | 187 | Type: progesterone IUD | 205 |
| Reason: heavy cycles | 187 | Type: vaginal ring | 205 |
| Reason: acne | 138 | Type: oral progestin | 205 |
| Reason: polycystic ovary syndrome | 187 | Type: hormone replacement therapy | 205 |
| Reason: ovarian cyst | 138 | Type: hormonal implant | 205 |
| Reason: others | 187 | Type: estrogen included | 205 |
| Emergency contraception (ever) | 136 | Type: progestin-only included | 205 |
| Number of treatment types used | 205 | Type: others | 205 |
| Type: combined birth control pill | 205 | Type: not known | 205 |

1.2. Biomarker protein levels in serum and plasma for cases and controls

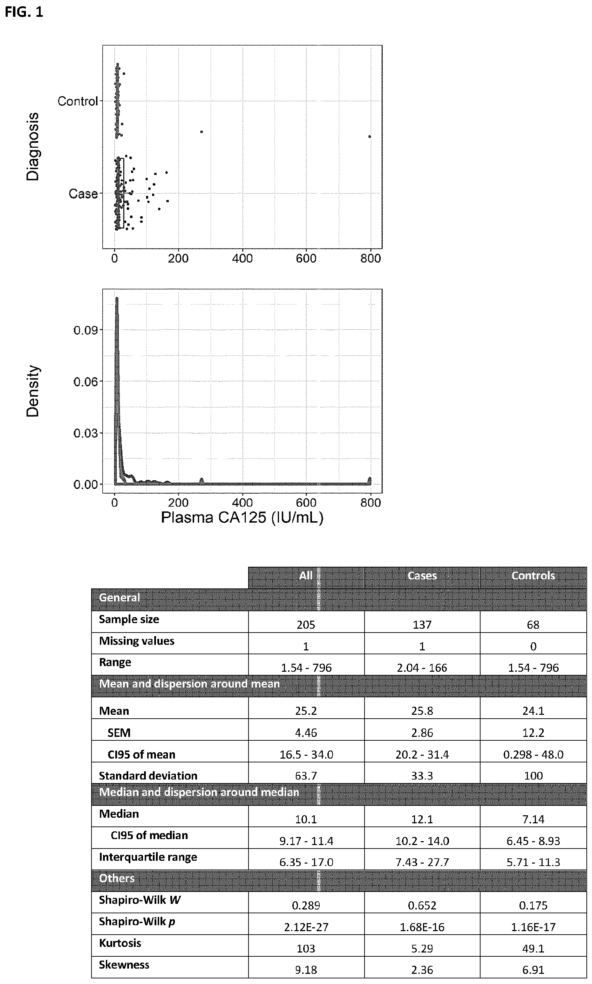

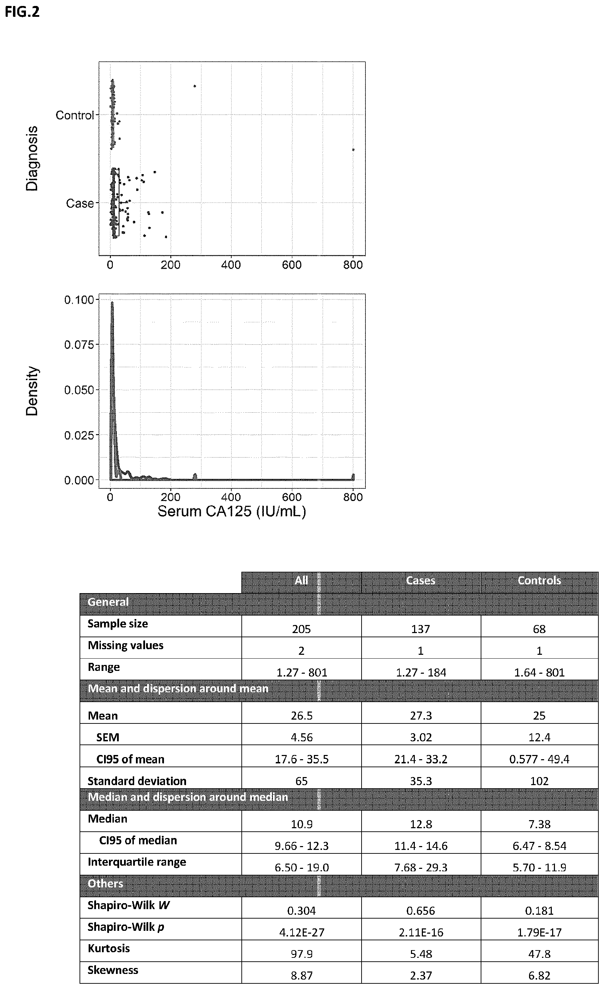

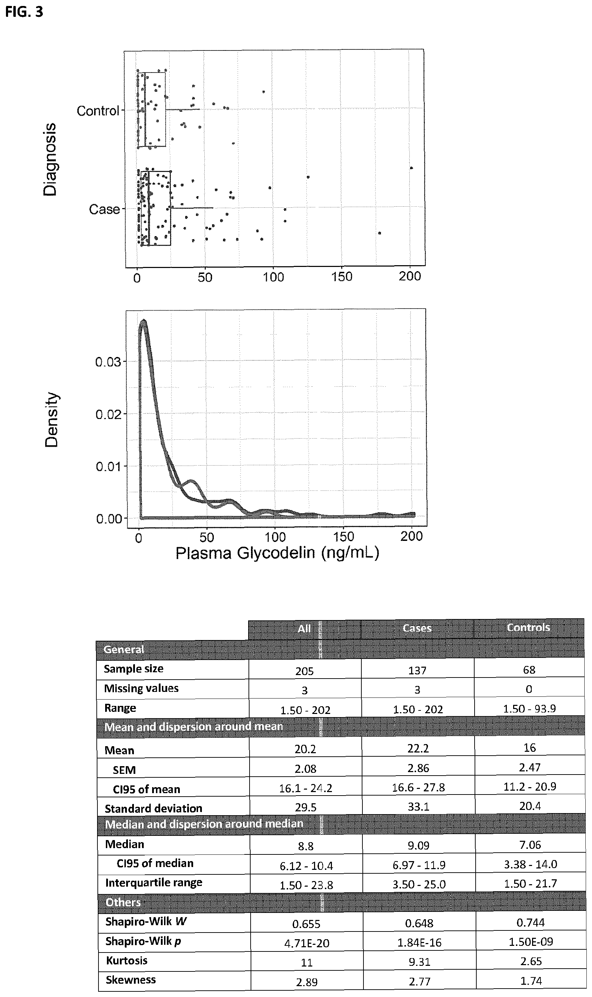

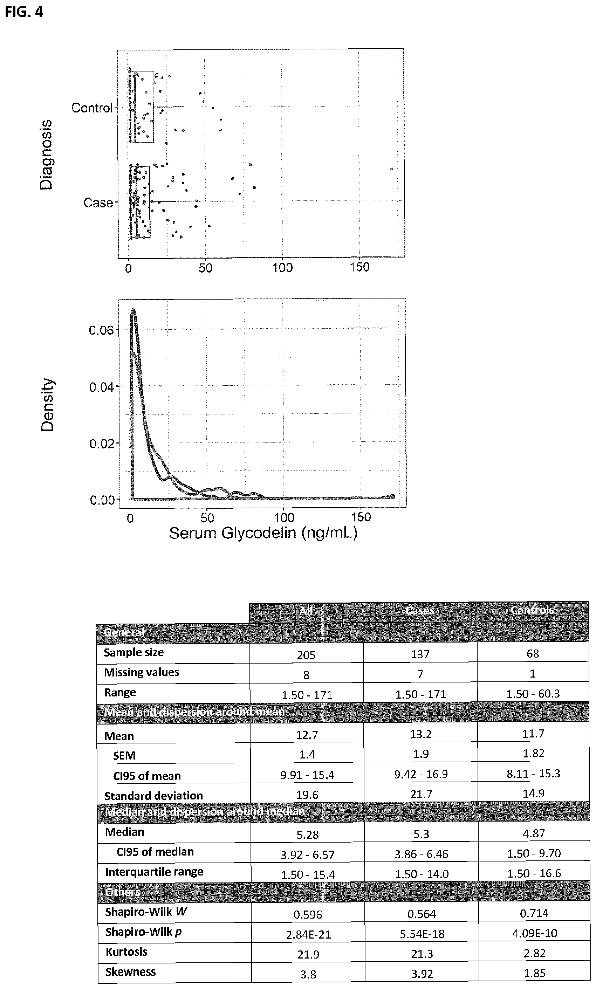

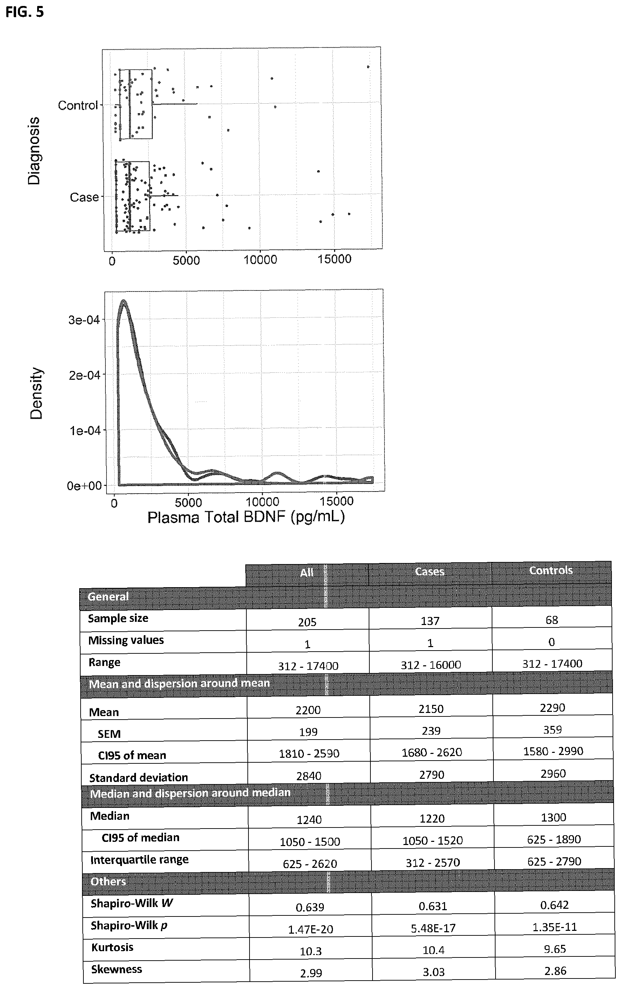

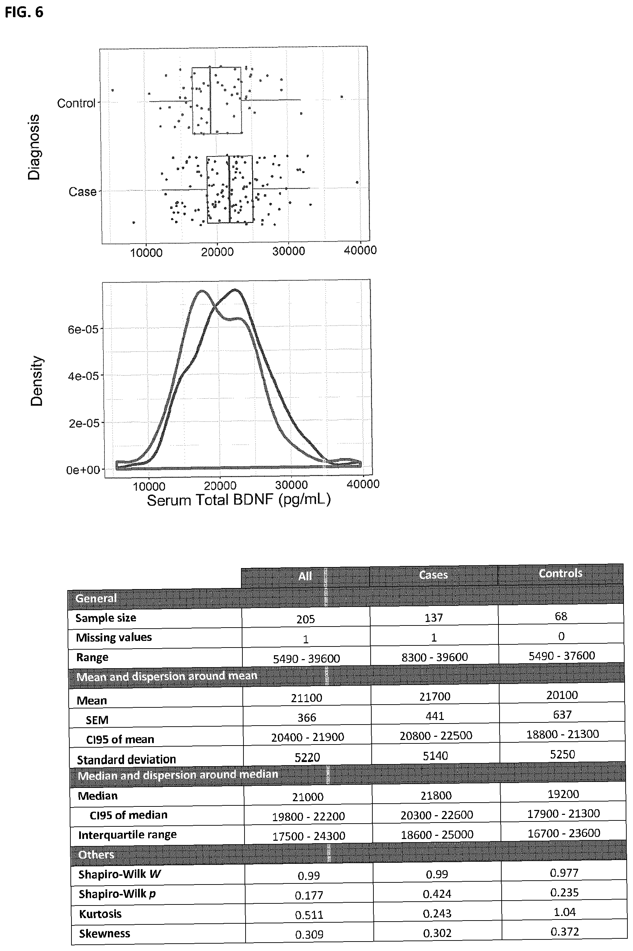

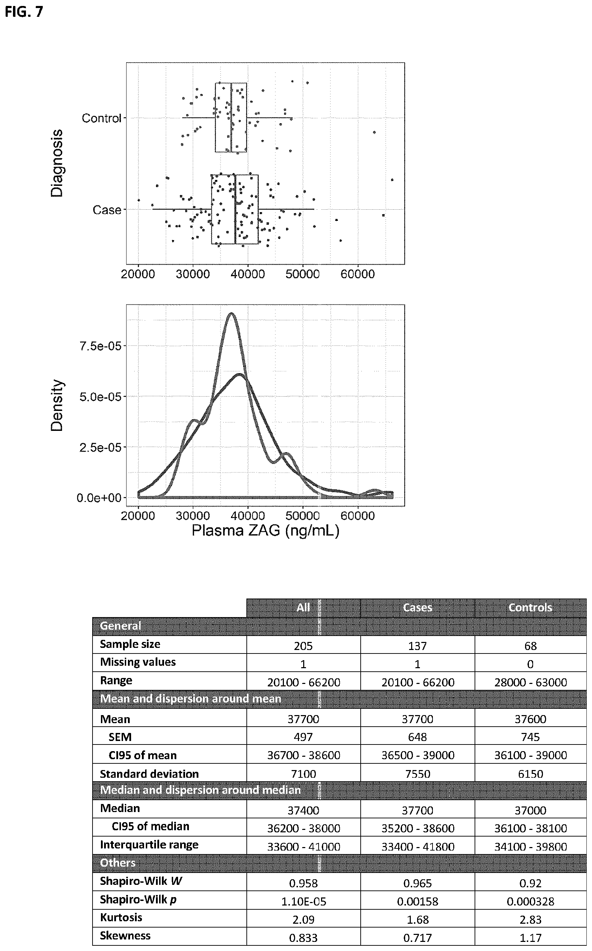

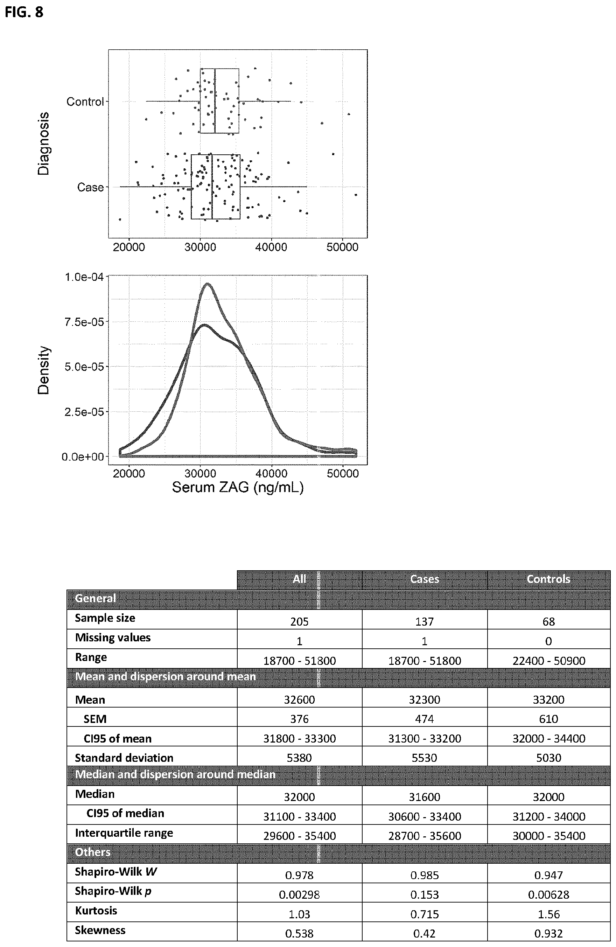

In FIGS. 1 to 8, all the biomarker data is summarized, grouped by the outcome of an endometriosis diagnostic evaluation (presence [case] of absence [control]).

1.3 Association studies

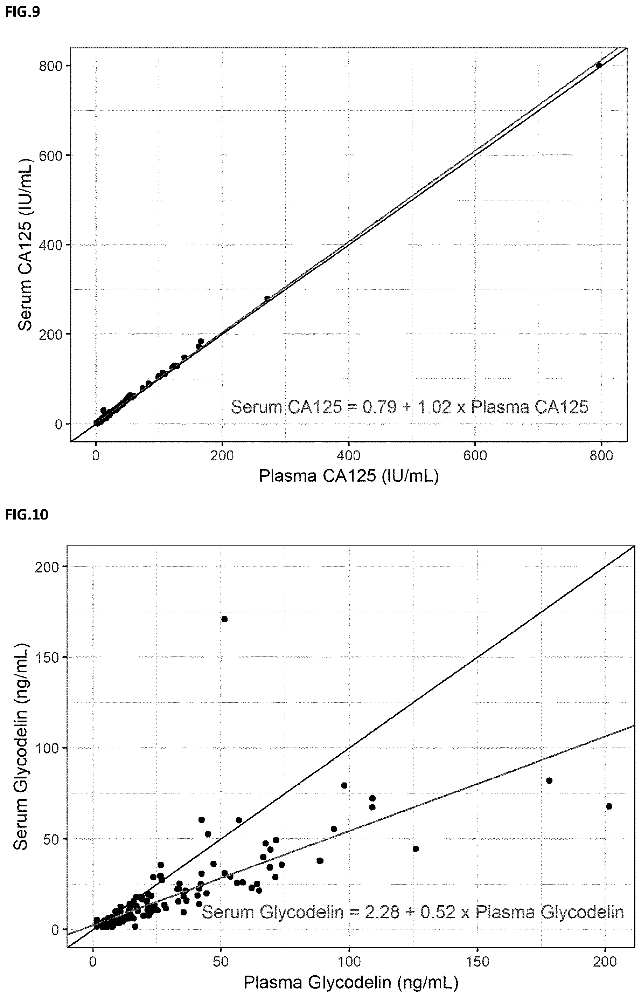

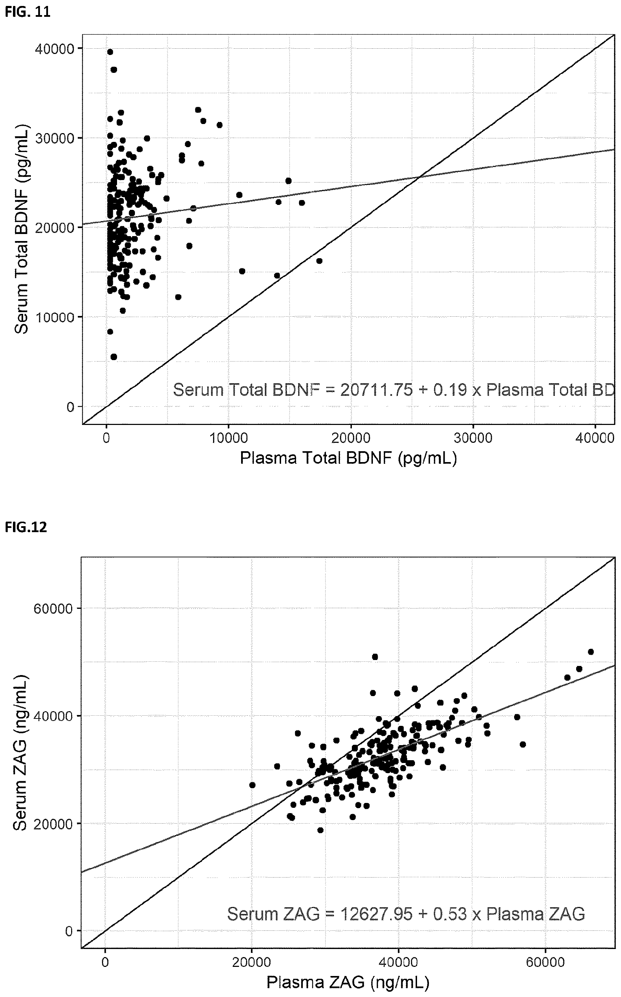

The association between the concentration of these 4 biomarkers in serum and plasma was assessed. The results are provided in FIGS. 9 to 12.

**Table 3. -Plasma versus serum biomarker levels**

| Analyte | Slope | 95% CI | Intercept | 95% CI | Adjusted $R^2$ | Pearson's correlation coefficient |
|---|---|---|---|---|---|---|
| CA125 | 1.02 | 1.01 - 1.02 | 0.787 | 0.446 - 1.13 | 0.999 | 0.999 |
| Glycodelin | 0.52 | 0.461 - 0.58 | 2.28 | 0.158 - 4.4 | 0.603 | 0.778 |
| Total BDNF | 0.192 | -0.06 - 0.444 | 20700 | 19800 - 21600 | 0.00603 | 0.105 |
| ZAG | 0.529 | 0.453 - 0.606 | 12600 | 9690 - 15600 | 0.476 | 0.692 |

The correlation between plasma and serum CA125 was found to be very high (0.999), indicating that the measurement of CA125 is unaffected by specimen type. For glycodelin and ZAG, the correlation was high (0.778 and 0.692, respectively), with values that tend to be lower in serum, especially towards the higher concentrations. In contrast to these markers, there was no correlation between total BDNF levels in serum and in plasma with a very low correlation coefficient (0.105). From these observations, it is clear that BDNF is most strongly affected by specimen type, either by specimen type-dependent differences in concentration or the presence of specimen type-specific factors that interfere with accuracy.

1.4 Statistical analysis of biomarkers in cases vs controls

**Mann-Whitney U test**

**Table 4. -Mann-Whitney U tests results comparing- quantitative markers between case and control groups.** The difference in location represents the estimated median of the differences between a case sample and a control sample. Parameters which were statistically significant (p value ≤ 0.05) are highlighted in bold.

| Analyte | Class 1 | Class 2 | *W* | *p* value | Difference in location |
|---|---|---|---|---|---|
| **Serum CA125** | Case | Control | 6342 | **5.72E-06** | 0.477 |
| **Plasma CA125** | Case | Control | 6339 | **1.62E-05** | 0.434 |
| **Serum Total BDNF** | Case | Control | 5450 | **0.0379** | 0.0794 |
| Plasma Glycodelin | Case | Control | 5039 | 0.214 | 0.0532 |
| Serum ZAG | Case | Control | 4196 | 0.282 | -0.0231 |
| Plasma Total BDNF | Case | Control | 4469 | 0.696 | -5.43E-05 |
| Plasma ZAG | Case | Control | 4764 | 0.726 | 0.00804 |
| Serum Glycodelin | Case | Control | 4407 | 0.89 | 7.06E-06 |

The endometriosis case group had a significantly higher mean level of serum CA125, plasma CA125 and serum total BDNF, and a higher age at onset of intercourse pain and age at surgery. There was no statistical difference between mean level of ZAG and Glycodelin (in plasma or serum) between cases and controls groups.

1.5 Univariate models for endometriosis diagnosis

The ROC analysis was applied to analyse different performance outcomes (AUC, optimal Youden's index, maximum accuracy, sensitivity at 95% specificity, specificity at 95% sensitivity) and identify those markers presenting a higher predictive value.

**Table 5. - Performance outcomes of each biomarker.** The AUC results of the 4 protein biomarkers in serum and plasma are highlighted in bold.

| Parameter | Youden's index | Sensitivity | Specificity | Accuracy | AUC |
|---|---|---|---|---|---|
| Serum CA125 | 0.356 (0.221 - 0.491) | 0.64 (0.552 - 0.719) | 0.716 (0.591 - 0.817) | 0.665 (0.595 - 0.729) | **0.696** (0.623 - 0.769) |
| Plasma CA125 | 0.338 (0.201 - 0.475) | 0.676 (0.59 - 0.753) | 0.662 (0.536 - 0.769) | 0.672 (0.602 - 0.735) | 0.685 (0.611 - 0.759) |
| Serum Total BDNF | 0.199 (0.06 - 0.337) | 0.757 (0.675 - 0.825) | 0.441 (0.323 - 0.566) | 0.652 (0.582 - 0.716) | **0.589** (0.508 - 0.67) |
| Plasma Total BDNF | 0.169 (0.061 - 0.277) | 0.287 (0.214 - 0.372) | 0.882 (0.776 - 0.944) | 0.485 (0.415 - 0.556) | **0.517** (0.433 - 0.60) |
| Serum ZAG | 0.147 (0.037 - 0.257) | 0.279 (0.208 - 0.364) | 0.868 (0.759 - 0.934) | 0.475 (0.406 - 0.546) | **0.546** (0.463 - 0.629) |
| Plasma Glycodelin | 0.136 (-0.001 - 0.273) | 0.754 (0.67 - 0.822) | 0.382 (0.27 - 0.509) | 0.629 (0.558 - 0.695) | 0.553 (0.47 - 0.636) |
| Plasma ZAG | 0.125 (-0.015 - 0.265) | 0.463 (0.378 - 0.551) | 0.662 (0.536 - 0.769) | 0.529 (0.459 - 0.599) | **0.515** (0.431 - 0.599) |
| Serum Glycodelin | 0.062 (-0.085 - 0.209) | 0.569 (0.48 - 0.655) | 0.493 (0.37 - 0.616) | 0.543 (0.471 - 0.614) | **0.506** (0.421 - 0.591) |

Serum/plasma CA125 and serum BDNF had the strongest diagnostic performance in segragating between cases and controls, based on the AUC value. ZAG and glycodelin showed a quiet lower performance. In agreement with the results of the statistical analysis shown above, the diagnostic perfomance of BDNF is significantly higher when BDNF is measured in serum than in plasma.

## Example 2.- Comparison between the United Kingdom (UK), Canada and Italy studies

The results for the four (4) biomarkers in the study in the UK population described under Example 1, were compared with samples from Italy (Ospedale San Rafaele)

## 2.1 MATERIALS AND METHODS (OSR)

Sample collection:

Data collection protocol and biological samples was performed under GWA endometriosis project. Blood samples were collected from patients admitted for endometriosis surgery and frozen immediately. All hematoclinical examinations along with filled questionaires were anonimized using patient's code. Data from patients with endometriosis (n = 119) and healthy controls (n = 60) were collected.

| Outcome | Stage | Frequency | Proportion |
|---|---|---|---|
| Control | 0 | 60 | 33.5% |
| Case | 1-2 | 20 | 11.2% |
| Case | 3-4 | 90 | 50.3% |
| Case | NA | 9 | 5.0% |

Sample analysis:

Data were analyzed at Laboratorio di Scienze Riproduttive Divisione di genetica e biologia cellulare. Mean concentration values of the biomarkers in the isolated biological samples from cases and controls were determined using commercial ELISA kits according to the manufacturer's instructions.

| Analyte | Kit | Catalogue number | Supplier |
|---|---|---|---|
| Total BDNF | BDNF Emax ® ImmunoAssay System | G7610 | Promega Milano |
| CA125 | Human CA125 Quantikine® ELISA | DCA125 | R&D Systems, Abingdon, UK |
| Glycodelin | Glycodelin ELISA | BD-30-20 | BIOSERV DIAGNOSTICS, Rostock, Germany |
| ZAG | Human ZAG ELISA kit | KA1689 | Abnova, Taiwan. |
| **ELISA kits used for total BDNF, CA125, Glycodelin and ZAG determination** | | | |

## 2.2. RESULTS

Performance comparison of individual biomarkers in the different populations

**CA125**

| Source | Youden | AUC |
|---|---|---|
| Italy (Serum) | 0.472 (0.346 - 0.597) | 0.785 (0.719 - 0.851) |
| United Kingdom (Plasma) | 0.338 (0.201 - 0.475) | 0.685 (0.611 - 0.759) |
| United Kingdom (Serum) | 0.356 (0.221 - 0.491) | 0.696 (0.623 - 0.769) |

**BDNF**

| Source | Youden | AUC |
|---|---|---|
| Italy (Serum) | 0.137 (-0.009 - 0.283) | 0.547 (0.459 - 0.635) |
| United Kingdom (Plasma, Total) | 0.169 (0.061 - 0.277) | 0.517 (0.433 - 0.601) |
| United Kingdom (Serum, Total) | 0.199 (0.06 - 0.337) | 0.589 (0.508 - 0.67) |

**Glycodelin (PP14)**

| Source | Youden | AUC |
|---|---|---|
| Italy (Serum) | 0.162 (0.014 - 0.31) | 0.558 (0.47 - 0.646) |
| United Kingdom (Plasma) | 0.136 (-0.001 - 0.273) | 0.553 (0.47 - 0.636) |
| United Kingdom (Serum) | 0.062 (-0.085 - 0.209) | 0.506 (0.421 - 0.591) |

**ZAG**

| Source | Youden | AUC |
|---|---|---|
| Italy (Serum) | 0.123 (0.027 - 0.218) | 0.524 (0.433 - 0.615) |
| United Kingdom (Plasma) | 0.125 (-0.015 - 0.265) | 0.515 (0.431 - 0.599) |

(continued)

| Source | Youden | AUC |
|---|---|---|
| United Kingdom (Serum) | 0.147 (0.037 - 0.257) | 0.546 (0.463 - 0.629) |

In univariate analysis, by far the best-performing biomarker was CA125 in the study in Italy (Ospedale San Raffaele), with an AUC of 0.785. The highest AUC for BDNF was in the UK (Oxford) study. The ROC data of glycodelin and ZAG in the UK and Italian populations of study do not justify their inclusion into the algorithm for the diagnosis of endometriosis.

**Example 3.- Identification of informative predictors for endometriosis diagnosis**

The data collected in the Oxford study discussed in Example 1 was further analysed with the goal of identifying quantitative and categorical predictors which may be used in an algorithm for the diagnosis of endometriosis.

**Material and methods**

The material and methods described below are similar to those described in example 1 (same sample, same materials, and same methods). Only BDNF and CA125 are quantified here, using the set of biomarkers that is included in EndomKIT. For completeness, we describe the method again below.

Definition of cases and control groups.

This prospective study was conducted on serum samples acquired from the Oxford Endometriosis CaRe Centre biobank (UK). The experimental protocol was approved by ethics committee CEIm HM Hospitales.

204 serum samples obtained from patients of reproductive age (18 - 50 years old) with suspicion of endometriosis in which the disease has been confirmed or excluded by histological evaluation of excised lesions during laparoscopy. Patients were pre-classified as controls and cases and anonymized in the biobank: Case/control classification was performed based on laparoscopy and histological verification of endometrial glands and/or stroma in the excised lesions. Patients were classified as cases if the endometriosis was confirmed by laparoscopy and histology, and as controls if endometriosis could not be confirmed by laparoscopy and histology. Patients were excluded if they had taken a hormonal treatment in the three months prior to blood extraction. Analysis was performed blinded to the surgical and imaging findings. The patients with endometriosis were stratified according to the revised American Society of Reproductive Medicine (rASRM) classification. The main characteristics of the patients included in this study are displayed in Table 1.

Table 1. Demographic characteristics of the patients in the Oxford Endometriosis CaRe

| | Controls N=68 | Endometriosis N=136 |
|---|---|---|
| Age years (mean $\pm$ SD) | 33.5 (5.96) | 35.6 (6.42) |
| BMI (mean $\pm$ SD) | 25.38 (4.63) | 26.46 (5.32) |
| rASRM classification | | |
| I-II | - | 68 (50%) |
| III-IV | - | 68 (50%) |
| Control diagnosis | | |
| Note. BMI= Body Mass Index; rASRM= revised American Society for Reproductive Medicine. | | |
| Centre biobank cohort1. | | |

Sample collection, preservation and storage

The specimens were collected and handled following the World Endometriosis Research Foundation Standard Operating procedures (Rahmioglu et al. 2014) after receiving patients' consent. Patients were asked to fast for at least 10 hours prior to blood collection. Serum samples were stored in a biobank at -80 °C for up to 5 years and were transferred to the laboratory analysis site.

Biomarker concentration determination.

Mean concentration values of the biomarkers in the isolated biological samples from serums and controls were determined using commercial ELISA kits according to the manufacturer's instructions.

Methods of statistical analysis

The non-parametric two-sided Mann-Whitney U test, also known as Wilcoxon rank-sum test, was implemented with the wilcox.test function from the stats package. The null hypothesis is that the distributions of the case predictor values, and the control predictor values differ by a location shift of 0 (no shift), with the alternative that they differ by a location shift different from 0. From the resulting W (or U) statistic, a p value is calculated. The difference in location represents the estimated median of the differences between a case sample and a control sample.

For each numerical (quantitative) variable, univariate logistic regression was performed using the glm function from the stats package on default settings (family = "binomial"). With the resulting model, the probabilities of diagnosis were calculated for each data point with the predict function, and the resulting dataframe was applied on the roc function from the pROC package to obtain sensitivity (TP/(TP + FN)) and specificity (TN/(TN + FP)) values across a range of threshold values. The true positive (TP), true negative (TN), false positive (FP) and false negative (FN) results were obtained, together with Youden's index (sensitivity + specificity - 1), accuracy ((TP + TN)/(TP + TN + FP + FN)), positive (sensitivity/(1 - specificity)) and negative likelihood ratio (specificity/(1-sensitivity)), diagnostic odds ratio ((TP x TN) / (FP x FN)), the positive (TP/(TP + FP)) and negative (TN/(TN + FN)) predictive values, according to the standard definitions. Predictive values were also calculated based on an estimated true prevalence of 24% or 50% for positive diagnosis of endometriosis:

$$tPPV = \frac{tPrev \times Sensitivity}{tPrev \times Sensitivity + (1 - tPrev) \times (1 - Specificity)}$$

$$tNPV = \frac{(1 - tPrev) \times Specificity}{(1 - tPrev) \times Specificity + tPrev \times (1 - Sensitivity)}$$

The receiver-operator characteristic (ROC) curve was plotted based on the true positive rate (sensitivity) and false positive rate (1 - specificity) along the spectrum of threshold values. The area under the ROC curve (AUC) was calculated using the auc function of the pROC package.

Confidence intervals for proportions were obtained with the binom.confint function from the binom package, or from applying the diagnostic function from the ThresholdROC package.

For tPPV and tNPV confidence interval estimations, the sample size was set at respectively TP + FP and TN + FN.

Chi-squared analysis was performed using the chisq.test function (stats package) on categorical variables to identify a pairwise statistically significant difference in diagnostic outcome between the categories. In case of ordered categorical data, the Cochran-Armitage test was used, based on function CochranArmitageTest from the DescTools package.

To evaluate for associations between predictors, chi-squared analysis (or Cochran-Armitage testing) was used when comparing two categorical variables, the Mann-Whitney U test when comparing a numerical with a categorical variable, and Pearson's correlation coefficient (cor function from stats package) when comparing two numerical variables.

Because of the large number of (non-independent) pairwise comparisons, the p values were adjusted using the Benjamini-Hochberg correction for analyses involving categorical variables (vs outcome, vs numerical variable, vs other categorical variable), with corrections for p values grouped by categorical parameter.

To assess whether both CA125 and BDNF are important contributors (with statistical significance) in a logistic regression model for endometriosis diagnosis, the glm function from the stats package was applied with log transformed CA125 and BDNF as input variables and endometriosis diagnosis as outcome. Next, the step function from the stats package was used at the default settings to evaluate whether both parameters contributed meaningfully to the model.

The glm function from the stats package was used to generate a logistic regression model without interaction terms with the selected input variables. After training, the models were tested on the isolated testing data using the predict function and subsequently, the roc function was applied to analyze different performance outcomes (AUC, optimal Youden's index, maximum accuracy, sensitivity at 95% specificity, specificity at 95% sensitivity).

The final model was created by including all individuals for development of a logistic regression model. The associated performance metrics values were also reported.

## Hormone treatment data

The following treatment types were considered to contain only progestin/progesterone: progestin-only birth control pill, progestin injection, vaginal ring (NuvaRing), progesterone-containing coil/IUD, hormonal implant (Implanon/-Nexplanon) and oral progestin. The following treatment types were considered to involve both estrogen and progestin: combined birth control pill and hormone replacement therapy.

## Severity of last menstrual pain

Values from 0 to 5 were grouped ("Pain 0-5"; "None/Mild") as well as the values from 6 to 10 ("Pain 6-10"; "Moderate/Severe").

## Frequency of harder stools in case of period pain in the last 3 months

For the frequency of harder stools in case of period pain in the last 3 months, the answers "Sometimes", "Often", "Most of the time" and "Always" were grouped into "Common" while the answer "Never/rarely" was renamed "Not common". All patients who received a questionnaire and who did not indicate the frequency are assumed to not have experienced any cases of harder stools.

## **Results**

3.1. Numerical predictors with statistically significant differences between endometriosis cases and controls

**Table: Mann-Whitney *U* test**

| Analyte | Size | Class 1 | Class 2 | *U* | *p* value | Difference in location |
|---|---|---|---|---|---|---|
| CA125 | 202 | Case | Control | 6582 | 2.41E-07 | 8.500 |
| Time between first endometriosis symptoms and surgery | 102 | Case | Control | 1473.5 | 0.0010 | 6.000 |
| BDNF | 204 | Case | Control | 5783.5 | 0.0035 | 2550 |
| Age at first experience of intercourse pain | 89 | Case | Control | 1201 | 0.0093 | 5.000 |
| Age at first experience of ovarian cyst symptoms | 49 | Case | Control | 334.5 | 0.0233 | 7.000 |
| Age (at time of surgery) | 204 | Case | Control | 5514.5 | 0.0249 | 2.000 |
| Age starting regular ibuprofen use | 53 | Case | Control | 343.5 | 0.0384 | 7.000 |
| Time between onset of period pain symptoms and surgery | 68 | Case | Control | 490.5 | 0.0386 | 6.000 |
| How old were you when you were diagnosed with endometriosis? | 75 | Case | Control | 516 | 0.0467 | 4.852 |

For both biomarkers CA125 and BDNF, a statistically significant difference was found between the endometriosis case and the control groups. Higher CA125 and BDNF levels were significantly associated with endometriosis ($p < 10^{-4}$ and $p = 0.0035$, respectively). The medians of some other numerical predictors were also significantly higher in the case group (all $p < 0.05$), all of them related to age.

3.2. Sensitivity/specificity analysis for biomarkers

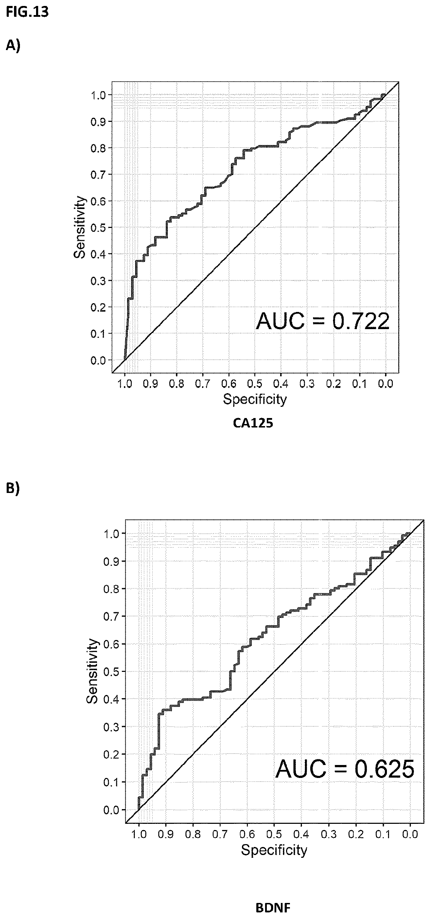

Receiver-operator characteristics (ROC) curves for CA125 and BDNF biomarkers are shown in Figures 13 A and B, respectively.

| Parameter | Youden's index | Sensitivity | Specificity | Accuracy | AUC |
|---|---|---|---|---|---|
| CA125 | 0.361 (0.237 - 0.485) | 0.537 (0.449 - 0.623) | 0.824 (0.708 - 0.902) | 0.634 (0.563 - 0.699) | 0.722 (0.652 - 0.792) |
| BDNF | 0.272 (0.167 - 0.377) | 0.360 (0.281 - 0.448) | 0.912 (0.811 - 0.964) | 0.544 (0.473 - 0.613) | 0.625 (0.547 - 0.704) |

The area-under-the-curve (AUC) values for CA125 and BDNF are 0.722 and 0.625, respectively, indicating moderate correlation with endometriosis. At the optimal cutoff value, as determined by Youden's index, a sensitivity of 53.7% was found for CA125, with a specificity of 82.4%. For BDNF, the sensitivity is 36.0% for a specificity of 91.2% at optimal Youden's index.

3.3. Categorical predictors with statistically significant differences between endometriosis cases and controls

**Chi-squared and Cochran-Armitage tests**

| Predictor | Test | *p* value (adjusted) | Size | Class | Case | Control | Case (%) | Control (%) |
|---|---|---|---|---|---|---|---|---|
| Previous surgery for endometriosis | Chi-square | 3.70E-07 | 196 | Yes | 71 | 7 | 54.6 | 10.6 |
| | | | | No | 59 | 59 | 45.4 | 89.4 |
| Previous diagnosis of endometriosis | Chi-square | 1.92E-06 | 139 | Yes | 67 | 12 | 74.4 | 24.5 |
| | | | | No | 23 | 37 | 25.6 | 75.5 |
| Painful periods as symptom of endometriosis | Chi-square | 7.00E-06 | 191 | Yes | 94 | 25 | 76.4 | 36.8 |
| | | | | No | 29 | 43 | 23.6 | 63.2 |
| Severity of last menstrual cycle pain | Chi-square | <0.01 * | 191 | None/ Mild | 27 | 36 | 22.0 | 52.9 |
| | | | | Moderate / Severe | 96 | 32 | 78.0 | 47.1 |
| Severity of menstrual cycle pain in general (scale) | Cochran-Armitage | 0.031 | 138 | Scale 1 | 0 | 2 | 0 | 4.1 |
| | | | | Scale 2 | 5 | 10 | 5.6 | 20.4 |
| | | | | Scale 3 | 31 | 18 | 34.8 | 36.7 |
| | | | | Scale 4 | 53 | 19 | 59.6 | 38.8 |
| Previous diagnosis of endometriosis by a gynecologist after surgery | Chi-square | 0.033 | 49 | Yes | 11 | 16 | 35.5 | 88.9 |
| | | | | No | 20 | 2 | 64.5 | 11.1 |
| Hormonal implant | Chi-square | 0.048 | 191 | Yes | 3 | 11 | 2.4 | 16.2 |
| | | | | No | 120 | 57 | 97.6 | 83.8 |
| Frequency of harder stools | Chi-square | <0.05** | 191 | Common | 46 | 9 | 37.4 | 13.2 |

(continued)

| Predictor | Test | *p* value (adjusted) | Size | Class | Case | Control | Case (%) | Control (%) |
|---|---|---|---|---|---|---|---|---|
| during period pain in last 3 months | | | | | | | | |
| | | | | Not common | 77 | 59 | 62.6 | 86.8 |

*This test was performed separately from the other tests. The unadjusted *p* value was 2.66E-05 which would put the Benjamini-Hochberg-adjusted *p* value below 0.01.
**This test was performed separately from the other tests. The unadjusted *p* value was 0.00077 which would put the Benjamini-Hochberg-adjusted *p* value below 0.05.

The table above shows that several patient parameters are associated with endometriosis status. Most significantly, a previous history of endometriosis or surgery to examine endometriosis is more common among patients tested positive for current endometriosis. Painful periods as a symptom of endometriosis is also associated with a positive diagnosis with strong statistical significance. Other parameters with a *p*-value below 0.05 are the severity of menstrual cycle pain in general, the severity of the last menstrual cycle pain, the use of hormonal implant and the frequency of harder stools during period pain in the last 3 months.

3.4. Selection of predictors for the diagnosis of endometriosis in multivariate analysis

Although missing data can be estimated by imputation, a threshold of 10% for each predictor was used as the maximum proportion of missing data for imputation. In addition, because many predictors have a time component, several of them showed a correlation with age at time of surgery, either based on Pearson's correlation coefficient (for numerical predictors) or through Mann-Whitney *U* (for binary categorical predictors) or Kruskal-Wallis (for multiclass categorical predictors) analysis. As a result, the predictors with a significant number of missing data points, with a significant correlation with age at time of surgery or a significant association with another candidate predictor (with a more significant association with endometriosis) were excluded for multivariate analysis:

| Analyte | Size | Correlation with age at time of surgery |
|---|---|---|
| Age at first experience of intercourse pain | 89 | 0.634 |
| Age at first experience of ovarian cyst symptoms | 49 | 0.857 |
| Age starting regular ibuprofen use | 53 | 0.547 |
| Time between onset of period pain symptoms and surgery | 68 | 0.508 |
| How old were you when you were diagnosed with endometriosis? | 75 | 0.762 |
| Time between first endometriosis symptom and surgery | 102 | 0.429 |
| **Analyte** | **Size** | **Mann-Whitney *U* (or Kruskal-Wallis) against other candidate predictors (*p* value)** |
| Previous diagnosis of endometriosis | 139 | 6.29E-13[1] |
| Severity of menstrual cycle pain in general | 138 | 0.00666[2] |
| Previous diagnosis of endometriosis by a gynecologist after surgery | 49 | 0.000195[1] |
| 1: Record of previous surgery for endometriosis; 2: Time between first symptoms and surgery; 3: Age at time of surgery | | |

Every participant in the study underwent surgery, a necessary component of algorithm development to provide the true clinical state of each participant. Given that all the algorithm input variables have been collected immediately before the planned surgery, they are all correlated with this event. In this respect, as a variable, age is defined as "age at the time of surgery". For the diagnostic algorithm under development, age should be understood to mean the age during which the individual is evaluated for a possible diagnosis of endometriosis, not necessarily followed by surgery.

The variable "painful periods as a symptom for planned surgery", is explicitly correlated to the planned surgery. Since the time component is assumed to affect all the variables in the same way, for the algorithm under development, this variable should be understood to mean the period pain which the individual is suffering from during her evaluation for a possible diagnosis of endometriosis, not necessarily followed by surgery. Therefore, this variable is rephased to "painful periods as one of the symptoms that have led to the current examination for endometriosis".

Of the remaining candidate variables, age, chronic pelvic pain as purpose for hormone treatment and severity of worst pain during last menstrual period were not considered based on previous evaluations with these parameters, which showed they were not part of the most optimal input variable combinations (data not shown). Based on all the above considerations, the following predictors were selected as candidate input variables for multivariate analysis for the diagnosis of endometriosis:

- Serum CA125
- Serum BDNF
- Record of previous surgery for endometriosis
- Painful periods as a symptom for endometriosis examination (planned surgery)
- Hormonal implant use
- Frequency of harder stools in case of period pain in the last 3 months.

In addition, the following parameters are of continued interest but lack the sample sizes to consider for the multivariate model (for the intercourse pain, ovarian cyst and ibuprofen parameters) or because it was not a relevant independent contributor in preliminary multivariate analyses (for the menstrual pain severity parameter):

- Age at first experience of intercourse pain
- Age at first experience of ovarian cyst symptoms
- Severity of menstrual pain last time (1-10)
- Starting age of ibuprofen use

These parameters may be assessed in future surveys, and the answers of such surveys may be used for updates of the multivariate diagnostic model.

**Example 4.-Combination of the CA-125 and BDNF in serum biomarkers with one or more of the selected clinical variables results in an improved sensitivity and diagnostic value.**

The inventors further conducted a ROC analysis to investigate the performance of CA125 and BDNF, alone (Table 6) and when combined with some of the clinical variables identified in Example 3 to be associated with presence of endometriosis.

As a way of example, the results of combining the CA125 and BDNF biomarkers determined in serum as described herein above together with two or more of the following variables related to the subject's history and/or clinical characteristics are provided in Table 7):

- record of previous surgery for endometriosis;
- painful periods as a symptom for endometriosis examination;
- Hormonal implant use; and
- Frequency of harder stools in case of period pain in the last 3 months.

The combination of the CA125 and BDNF biomarkers with at least two of the clinical variables identified by the inventors as being significantly associated with endometriosis in Example 3 significantly increased the sensibility and the diagnostic performance of the diagnosis test in terms of AUC.

**Table 6: performance of the model combining CA125 and BDNF**

| Model based on biomarkers (CA125, BDNF) | | | | | |
|---|---|---|---|---|---|
| | Sensitivity | Specificity | Youden index | Accuracy | AUC |
| Specificity > 0.9 | 0.471 (0.385 - 0.558) | 0.912 (0.811 - 0.964) | 0.382 (0.275 - 0.49) | 0.618 (0.547 - 0.684) | 0.745 (0.678 - 0.812) |

(continued)

| Model based on biomarkers (CA125, BDNF) | | | | | |
|---|---|---|---|---|---|
| | Sensitivity | Specificity | Youden index | Accuracy | AUC |
| Specificity > 0.95 | **0.397** (0.315 - 0.485) | **0.956** (0.868 - 0.989) | 0.353 (0.257 - 0.449) | 0.583 (0.512 - 0.651) | **0.745** (0.678 - 0.812) |
| Maximum Youden's index | 0.551 (0.464 - 0.636) | 0.868 (0.759 - 0.934) | 0.419 (0.303 - 0.535) | 0.657 (0.587 - 0.721) | 0.745 (0.678 - 0.812) |

**Table 7: performance of a model combining CA125 and BDNF with a set of 2 or more of the above selected clinical variables.**

| Model based on biomarkers (CA125, BDNF), Surgery, Hormonal implant | | | | | |
|---|---|---|---|---|---|
| | Sensitivity | Specificity | Youden index | Accuracy | AUC |
| Specificity > 0.9 | 0.669 (0.583 - 0.746) | 0.912 (0.811 - 0.964) | 0.581 (0.477 - 0.685) | 0.75 (0.684 - 0.807) | 0.842 (0.789 - 0.895) |
| Specificity > 0.95 | **0.566** (0.479 - 0.65) | **0.956** (0.868 - 0.989) | 0.522 (0.426 - 0.619) | 0.696 (0.627 - 0.757) | **0.842** (0.789 - 0.895) |
| Maximum Youden's index | 0.713 (0.628 - 0.786) | 0.897 (0.793 - 0.954) | 0.61 (0.505 - 0.715) | 0.775 (0.71 - 0.829) | 0.842 (0.789 - 0.895) |

| Model based on biomarkers (CA125, BDNF), Surgery, Harder stool frequency | | | | | |
|---|---|---|---|---|---|
| | Sensitivity | Specificity | Youden index | Accuracy | AUC |
| Specificity > 0.9 | 0.654 (0.567 - 0.732) | 0.912 (0.811 - 0.964) | 0.566 (0.462 - 0.671) | 0.74 (0.673 - 0.798) | 0.832 (0.778 - 0.886) |
| Specificity > 0.95 | **0.566** (0.479 - 0.65) | **0.956** (0.868 - 0.989) | 0.522 (0.426 - 0.619) | 0.696 (0.627 - 0.757) | **0.832 (0.778 - 0.**886) |
| Maximum Youden's index | 0.743 (0.659 - 0.812) | 0.882 (0.776 - 0.944) | 0.625 (0.519 - 0.731) | 0.789 (0.726 - 0.842) | 0.832 (0.778 - 0.886) |

| Model based on biomarkers (CA125, BDNF), Painful periods, Hormonal implant | | | | | |
|---|---|---|---|---|---|
| | Sensitivity | Specificity | Youden index | Accuracy | AUC |
| Specificity > 0.9 | 0.559 (0.471 - 0.643) | 0.912 (0.811 - 0.964) | 0.471 (0.363 - 0.578) | 0.676 (0.607 - 0.739) | 0.849 (0.798 - 0.900) |
| Specificity > 0.95 | **0.529** (0.442 - 0.615) | **0.956** (0.868 - 0.989) | 0.485 (0.388 - 0.582) | 0.672 (0.602 - 0.735) | **0.849** (0.798 - 0.900) |
| Maximum Youden's index | 0.816 (0.739 - 0.875) | 0.794 (0.675 - 0.879) | 0.61 (0.494 - 0.726) | 0.809 (0.747 - 0.859) | 0.849 (0.798 - 0.900) |

| Model based on biomarkers (CA125, BDNF), Painful periods, Harder stool frequency | | | | | |
|---|---|---|---|---|---|
| | Sensitivity | Specificity | Youden index | Accuracy | AUC |
| Specificity > 0.9 | 0.559 (0.471 - 0.643) | 0.912 (0.811 - 0.964) | 0.471 (0.363 - 0.578) | 0.676 (0.607 - 0.739) | 0.840 (0.787 - 0.893) |
| Specificity > 0.95 | **0.375** (0.295 - 0.462) | **0.956** (0.868 - 0.989) | 0.331 (0.236 - 0.426) | 0.569 (0.498 - 0.637) | **0.840** (0.787 - 0.893) |
| Maximum Youden's index | 0.86 (0.788 - 0.912) | 0.706 (0.581 - 0.807) | 0.566 (0.443 - 0.689) | 0.809 (0.747 - 0.859) | 0.840 (0.787 - 0.893) |

| Model based on biomarkers (CA125, BDNF), Hormonal implant, Harder stool frequency | | | | | |
|---|---|---|---|---|---|
| | Sensitivity | Specificity | Youden index | Accuracy | AUC |
| Specificity > 0.9 | 0.515 (0.428 - 0.601) | 0.912 (0.811 - 0.964) | 0.426 (0.319 - 0.534) | 0.647 (0.577 - 0.712) | 0.803 (0.744 - 0.862) |

(continued)

**Model based on biomarkers (CA125, BDNF), Hormonal implant, Harder stool frequency**

|  | Sensitivity | Specificity | Youden index | Accuracy | AUC |
|---|---|---|---|---|---|
| Specificity > 0.95 | **0.441** (0.357 - 0.529) | **0.956** (0.868 - 0.989) | 0.397 (0.3 - 0.494) | 0.613 (0.542 - 0.679) | **0.803** (0.744 - 0.862) |
| Maximum Youden's index | 0.706 (0.621 - 0.779) | 0.794 (0.675 - 0.879) | 0.5 (0.377 - 0.623) | 0.735 (0.668 - 0.793) | 0.803 (0.744 - 0.862) |

**Model based on biomarkers (CA125, BDNF), Surgery, Painful periods**

|  | Sensitivity | Specificity | Youden index | Accuracy | AUC |
|---|---|---|---|---|---|
| Specificity > 0.9 | 0.603 (0.515 - 0.685) | 0.912 (0.811 - 0.964) | 0.515 (0.408 - 0.621) | 0.706 (0.637 - 0.766) | 0.867 (0.819 - 0.915) |
| Specificity > 0.95 | **0.515** (0.428 - 0.601) | 0.956 (0.868 - 0.989) | 0.471 (0.373 - 0.568) | 0.662 (0.592 - 0.725) | **0.867** (0.819 - 0.915) |
| Maximum Youden's index | 0.882 (0.813 - 0.929) | 0.706 (0.581 - 0.807) | 0.588 (0.467 - 0.709) | 0.824 (0.763 - 0.872) | 0.867 (0.819 - 0.915) |

**Model based on biomarkers (CA125, BDNF), Surgery, Hormonal implant, Painful periods, Harder stool frequency**

|  | Sensitivity | Specificity | Youden index | Accuracy | AUC |
|---|---|---|---|---|---|
| Specificity > 0.9 | 0.669 (0.583 - 0.746) | 0.912 (0.811 - 0.964) | 0.581 (0.477 - 0.685) | 0.75 (0.684 - 0.807) | 0.883 (0.838 - 0.928) |
| Specificity > 0.95 | **0.632** (0.545 - 0.712) | **0.956** (0.868 - 0.989) | 0.588 (0.494 - 0.683) | 0.74 (0.673 - 0.798) | **0.883** (0.838 - 0.928) |
| Maximum Youden's index | 0.897 (0.83 - 0.94) | 0.721 (0.597 - 0.819) | 0.618 (0.499 - 0.736) | 0.838 (0.779 - 0.885) | 0.883 (0.838 - 0.928) |

## References

- Johnson N.P. (2013). Consensus on current Management of endometriosis. Hum. Reprod. 2013, 28(6), 1552-1568.
- Zondervan, K.T. (2018). Endometriosis. Nat. Rev. Dis. Primer 2018, 4, 9.
- Zondervan, K.T. (2020). Endometriosis. N. Engl. J. Med. 2020, 382, 1244-1256.
- Vigano P. (2004). Endometriosis: epidemiology and aetiological factors. Best Pract. Res. Clin. Obstet. Gynaecol. 2004, 18:177-200.
- Somigliana E. (2006). Association between endometriosis and cancer: a comprehensive review and a critical analysis of clinical and epidemiological evidence. Gynecol. Oncol 2006, 101(2): 331-341
- Sinaii N. (2002). High rates of autoimmune and endocrine disorders, fibromyalgia, chronic fatigue syndrome and atopic diseases among women with endometriosis: a survey analysis. Hum. Reprod. 2002, 17(10), 2715-2724.
- Agarwal, S. K., Chapron, C., Giudice, L. C., Laufer, M. R., Leyland, N., Missmer, S. A., ... & Taylor, H. S. (2019). Clinical diagnosis of endometriosis: a call to action. American journal of obstetrics and gynecology, 220(4), 354-e1.
- Becker, C. (2022) ESHRE guideline: endometriosis. Human Reproduction Open, 2022, 2, 1-26.
- Dunselman, G.J. (2014). ESHRE guideline: management of women with endometriosis. Hum. Reprod. Oxf. Engl. 2014, 29, 400-412 (2014).
- Ling, F.W. (1999) Randomized controlled trial of depot leuprolide in patients with chronic pelvic pain and clinically suspected endometriosis. Pelvic Pain Study Group. Obstet. Gynecol. 1999, 93, 51-58.
- Jenkins, T.R (2008). Does response to hormonal therapy predict presence or absence of endometriosis? J. Minim. Invasive Gynecol. 2008, 15, 82-86.
- Ahn SH, Singh V, Tayade C. Biomarkers in endometriosis: challenges and opportunities. Fertil Steril. 2017;107(3):523-532.
- Nisenblat, V. (2016a) Combination of the non-invasive tests for the diagnosis of endometriosis. Cochrane Database Syst. Rev. 2016, 7, CD012281.

**EP 4 616 199 B1**

- Nisenblat, V. (2016b) Blood biomarkers for the non-invasive diagnosis of endometriosis. Cochrane Database Syst. Rev. 2016, 5, Art. No.: CD012179.
- Polacchini A, et al. (2015) A method for reproducible measurements of serum BDNF: comparison of the performance of six commercial assays. Sci Rep. 2015 Dec 10;5:17989.
- Lee SY, Koo YJ, Lee DH. Classification of endometriosis. Yeungnam Univ J Med. 2021 Jan;38(1):10-18
- Keckstein J, Saridogan E, Ulrich UA, Sillem M, Oppelt P, Schweppe KW, Krentel H, Janschek E, Exacoustos C, Malzoni M, Mueller M, Roman H, Condous G, Forman A, Jansen FW, Bokor A, Simedrea V, Hudelist G. The #Enzian classification: A comprehensive non-invasive and surgical description system for endometriosis. Acta Obstet Gynecol Scand. 2021 Jul;100(7):1165-1175.
- Capezzuoli et al.: Classification/staging systems for endometriosis: the state of the art; Gynecological and Reproductive Endocrinology and Metabolism 2020; 1(1):14-22.
- Kitawaki J, Ishihara H, Koshiba H, Kiyomizu M, Teramoto M, Kitaoka Y, Honjo H. Usefulness and limits of CA-125 in diagnosis of endometriosis without associated ovarian endometriomas. Hum Reprod. 2005 Jul;20(7):1999-2003
- Barbieri RL, Niloff JM, Bast RC Jr, Scaetzl E, Kistner RW and Knapp RC (1986) Elevated serum concentrations of CA-125 in patients with advanced endometriosis. Fertil Steril 45,630-634.

**Claims**

1. A method for the screening, diagnosis or monitoring of endometriosis or the presence of endometriosis type lesions in a female subject, wherein said method comprises:

    a) determining the protein levels of:

        i. cancer antigen 125 (CA125) in a blood, plasma or serum sample isolated from the subject, and
        ii. brain-derived neurotrophic factor (BDNF) in a serum sample isolated from the subject; and

    b) optionally, calculating a score from the protein levels determined in step a), optionally in combination with other gene or protein markers, biochemical parameters and/or variables related to the subject's history and/or clinical characteristics;
    c) comparing the protein levels of CA125 and BDNF in the subject's sample as determined in step a) with a corresponding reference value; or comparing the score obtained in b) with a reference value; wherein when the CA125 and BDNF protein levels in a), or the score in b), are increased with respect to the respective reference value is indicative of endometriosis or presence of endometriosis type lesions.

2. The method of claim 1, wherein said subject is **characterized by** or has been preselected for having signs and/or symptoms of endometriosis, preferably for having chronic pelvic pain and/or infertility.

3. The method of any of claims 1 or 2, wherein said subject is not pregnant, nor suffers from any other inflammatory disease.

4. The method of any of claims 1 to 3, wherein BDNF protein levels comprise proBDNF and mature BDNF (mBDNF) levels, the so-called Total BDNF levels.

5. The method of any of claims 1 to 4, wherein the determination of BDNF and CA125 levels is carried out by means of an immunoassay, preferably an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay, including any multiplex versions of any of the above.

6. The method of any of claims 1 to 5, wherein said immunoassay is an ELISA assay or any multiplex version thereof.

7. The method of claim 6, wherein said ELISA assay is a sandwich ELISA and comprises:

    a. a capture antibody; and
    b. a detection antibody, wherein the detection antibody is conjugated to a detection tag, such as an enzyme or biotin.

8. The method of any of claims 1 to 7, wherein for the calculation of the score in step b), the protein levels of BDNF and CA125 are used as variables, in combination with one, two, three, four, five, six, seven or eight of the variables related to the subject's characteristics selected from the group consisting of:

    a) a history of surgery for endometriosis diagnosis;
    b) painful periods as a symptom for endometriosis examination;
    c) hormonal implant use; and
    d) frequency of harder stools in case of period pain in the last 3 months;
    e) age at first experience of intercourse pain;
    f) age at first experience of ovarian cyst symptoms;
    g) severity of menstrual pain last time (1-10); and
    h) starting age of ibuprofen use.

9. The method of claim 8, wherein said variables are one, two, three or four selected from the group consisting of:

    a) a history of surgery for endometriosis diagnosis;
    b) painful periods as a symptom for endometriosis examination;
    c) hormonal implant use; and
    d) frequency of harder stools in case of period pain in the last 3 months.

10. The method of any of claims 1 to 9, wherein said subject is a human female subject.

11. The method of any of claims 1 to 10, wherein said method further comprises conducting a confirmatory test, such as a histologic confirmation after laparoscopy.

12. A computer implemented (CI) method for the screening, diagnosing or monitoring of endometriosis in a subject, preferably a female subject, wherein said method comprises:

    i. receiving by a computer program the information on the protein levels of a group of biomarkers in a serum sample isolated from the subject, wherein said biomarkers consist of BDNF and CA125, and optionally information on other gene or protein markers, biochemical parameters and/or variables related to the subject's history and/or clinical characteristics;
    ii. processing the information received according to step i) by conducting step c), and optionally step b), according to any of claims 1 to 11;
    iii. generate an output information, for instance through a terminal display, identifying or classifying a subject having or with a high likelihood to have endometriosis or as an individual who could benefit from an exploratory test, such as a laparoscopy.

13. A computer program comprising instructions which, when the program is executed by a computer, cause the computer to carry out steps ii) and/or iii), of the method of claim 12.

14. A data-processing apparatus comprising means for carrying out steps ii) and/or iii) of the method of claim 12.

15. In vitro use of a kit suitable for determining the protein levels of BDNF and CA125 in a serum sample isolated from a subject, in a method for screening, diagnosing or monitoring of endometriosis or the presence of endometriosis type lesions in a subject according to any of claims 1 to 11; wherein said kit comprises:

    i. a reagent for the quantification of the BDNF protein levels;
    ii. a reagent for the quantification of the CA125 protein levels;
    iii. optionally, a recombinant human BDNF and a recombinant human CA125 for the calibrator and the positive controls;
    iv. optionally, further comprising instructions for the use of said reagents in determining the protein levels of BDNF and CA125 in a serum sample isolated from a subject.

16. The use of a kit according to claim 15, wherein said kit is a sandwich ELISA kit which comprises:

    i. an anti-BDNF capture antibody and an anti-CA125 capture antibody;
    ii. an anti-BDNF detection antibody and an anti-CA125 detection antibody, wherein the detection antibody is conjugated to a detection tag, such as an enzyme (e.g., peroxidase or alkaline phosphatase) or biotin;

iii. an enzyme substrate, such as tetramethylbenzidine (TMBD);

iv. when the detection antibody is conjugated to biotin, an enzyme conjugated streptavidin;

v. optionally, a recombinant human BDNF and a recombinant human CA125 for the calibrator and the positive controls;

vi. optionally, further comprising instructions for the use of said reagents in determining the protein levels of BDNF and CA125 in a serum sample isolated from a female subject.

**Patentansprüche**

1. Verfahren zum Screening, zur Diagnose oder zur Überwachung von Endometriose oder der Anwesenheit von endometrioseartigen Verletzungen in einem weiblichen Individuum, wobei das genannte Verfahren Folgendes umfasst:

   a) das Bestimmen der Proteinniveaus von:

   i. Krebsantigen 125 (CA125) in einer Blut-, Plasma- oder Serumprobe, welche aus dem Individuum isoliert wurde, und

   ii. vom Gehirn stammenden neurotrophen Faktor (BDNF) in einer Serumprobe, welche aus dem Individuum isoliert wurde; und

   b) wahlweise, das Berechnen einer Punktzahl aus den Proteinniveaus, welche in Schritt a) bestimmt wurden, wahlweise in Kombination mit anderen Gen- oder Proteinmarkern, biochemischen Parametern und/oder Variablen bezüglich der Vorgeschichte und/oder klinischer Merkmale des Individuums;

   c) das Vergleichen der Proteinniveaus von CA125 und BDNF in der Probe des Individuums, wie in Schritt a) bestimmt wurden, mit einem entsprechenden Referenzwert; oder das Vergleichen der in b) erhaltenen Punktzahl mit einem Referenzwert; wobei, wenn die CA125- und BDNF-Proteinniveaus in a), oder die Punktzahl in b), in Bezug auf den jeweiligen Referenzwert erhöht sind, dies auf Endometriose oder die Anwesenheit von endometrioseartigen Verletzungen schließen lässt.

2. Verfahren nach Anspruch 1, wobei das genannte Individuum **dadurch gekennzeichnet** ist oder deswegen vorgewählt worden ist, dass es Zeichen und/oder Symptome von Endometriose aufweist, vorzugsweise dass es chronische Beckenschmerzen und/oder Unfruchtbarkeit aufweist.

3. Verfahren nach einem der Ansprüche 1 oder 2, wobei das genannte Individuum weder schwanger ist noch unter einer anderen entzündlichen Erkrankung leidet.

4. Verfahren nach einem der Ansprüche 1 bis 3, wobei die BDNF-Proteinniveaus proBDNF- und reife BDNF (mBDNF)-Niveaus, die sogenannten gesamt-BDNF-Niveaus, umfassen.

5. Verfahren nach einem der Ansprüche 1 bis 4, wobei die Bestimmung von BDNF- und CA125-Niveaus mittels eines Immunassays, vorzugsweise eines enzymverbundenen Immun-Sorptions-Versuches (ELISA), eines fluoreszenten Immun-Sorptions-Versuches (FIA), eines Chemolumineszenz-Immunassays (CIA), oder eines Radioimmuntests (RIA), eines enzymverstärkten Immunassays, eines Festphasenradioimmuntests (SPROA), eines Fluoreszenzpolarisations- (FP) Tests, eines Fluoreszenz-Resonanzenergietransfer- (FRET) Tests, eines zeitaufgelösten Fluoreszenz-Resonanzenergietransfer- (TR-FRET) Tests, eines Oberflächenplasmonresonanz- (SPR) Tests, einschließlich irgendeiner Multiplex-Version einer der oben genannten, vorgenommen wird.

6. Verfahren nach einem der Ansprüche 1 bis 5, wobei der genannte Immunassay ein ELISA-Test oder irgendeine Multiplex-Version desselben ist.

7. Verfahren nach Anspruch 6, wobei der genannte ELISA-Test ein Sandwich-ELISA ist, und Folgendes umfasst:

   a. einen Fangantikörper; und
   b. einen Detektionsantikörper, wobei der Detektionsantikörper mit einem Detektions-Tag, wie einem Enzym oder Biotin, konjugiert ist.

8. Verfahren nach einem der Ansprüche 1 bis 7, wobei für die Berechnung der Punktzahl in Schritt b), die Proteinniveaus von BDNF und CA125 als Variablen verwendet werden, in Kombination mit einem, zwei, drei, vier, fünf, sechs, sieben oder acht der mit den Merkmalen des Individuums verbundenen Variablen, ausgewählt aus der Gruppe bestehend aus:

a) einer Vorgeschichte von Chirurgie zur Diagnose von Endometriose;
b) schmerzhaften Zeitabschnitten als Symptom zur Untersuchung von Endometriose;
c) der Verwendung von Hormonimplantaten; und
d) der Frequenz von härterem Stuhl im Falle von Menstruationsschmerzen in den letzten 3 Monaten;
e) dem Alter beim ersten Erlebnis von Schmerzen beim Geschlechtsverkehr;
f) dem Alter beim ersten Erlebnis von Eierstockzystensymptomen;
g) dem Schweregrad von Menstruationsschmerzen beim letzten Mal (1-10); und
h) dem Anfangsalter der Verwendung von Ibuprofen.

9. Verfahren nach Anspruch 8, wobei die genannten Variablen eine, zwei, drei oder vier sind, ausgewählt aus der Gruppe bestehend aus:

a) einer Vorgeschichte von Chirurgie zur Diagnose von Endometriose;
b) schmerzhaften Zeitabschnitten als Symptom zur Untersuchung von Endometriose;
c) der Verwendung von Hormonimplantaten; und
d) der Frequenz von härterem Stuhl im Falle von Menstruationsschmerzen in den letzten 3 Monaten.

10. Verfahren nach einem der Ansprüche 1 bis 9, wobei das genannte Individuum ein menschliches weibliches Individuum ist.

11. Verfahren nach einem der Ansprüche 1 bis 10, wobei das genannte Verfahren zusätzlich das Vornehmen eines Bestätigungstests, wie einer histologischen Bestätigung nach Laparoskopie, umfasst.

12. Computerimplementiertes (CI) Verfahren zum Screening, zur Diagnose oder zur Überwachung von Endometriose in einem Individuum, vorzugsweise einem weiblichen Individuum, wobei das genannte Verfahren Folgendes umfasst:

i. das Empfangen mittels eines Computerprogramms der Information über die Proteinniveaus einer Gruppe von Biomarkern in einer Serumprobe, welche aus dem Individuum isoliert wurde, wobei die genannten Biomarker aus BDNF und CA125 bestehen, und wahlweise Information über andere Gen- oder Proteinmarker, biochemische Parameter und/oder Variablen bezüglich der Vorgeschichte und/oder klinischer Merkmale des Individuums;
ii. das Verarbeiten der gemäß Schritt i) empfangenen Information, indem Schritt c), und wahlweise Schritt b), nach einem der Ansprüche 1 bis 11 vorgenommen wird;
iii. das Erzeugen einer Ausgangsinformation, zum Beispiel über eine Terminalanzeige, unter Identifizierung oder Einstufung eines Individuums als Endometriose aufweisend oder mit einer hohen Wahrscheinlichkeit Endometriose aufzuweisen oder als Individuum, welches von einem Explorationstest, wie einer Laparoskopie, profitieren könnte.

13. Computerprogramm umfassend Anweisungen, welche, wenn das Programm von einem Computer ausgeführt wird, dem Computer dazu bringen, die Schritte ii) und/oder iii) des Verfahrens nach Anspruch 12 vorzunehmen.

14. Datenverarbeitungsgerät umfassend Mittel zum Vornehmen der Schritte ii) und/oder iii) des Verfahrens nach Anspruch 12.

15. *In-vitro-Verwendung* eines Kits, welches zur Bestimmung der Proteinniveaus von BDNF und CA125 in einer Serumprobe, welche aus einem Individuum isoliert wurde, geeignet ist, in einem Verfahren zum Screening, zur Diagnose oder zur Überwachung von Endometriose oder der Anwesenheit von endometrioseartigen Verletzungen in einem Individuum nach einem der Ansprüche 1 bis 11; wobei das genannte Kit Folgendes umfasst:

i. ein Reagenz zur Quantifizierung der BDNF-Proteinniveaus;
ii. ein Reagenz zur Quantifizierung der CA125-Proteinniveaus;
iii. wahlweise, einen rekombinanten menschlichen BDNF und einen rekombinanten menschlichen CA125 für den Kalibrator und die Positivkontrollen;
iv. wahlweise, zusätzlich umfassend Anweisungen für die Verwendung der genannten Reagenzien bei der Bestimmung der Proteinniveaus von BDNF und CA125 in einer Serumprobe, welche aus einem Individuum isoliert wurde.

16. Verwendung eines Kits nach Anspruch 15, wobei das genannte Kit ein Sandwich-ELISA-Kit ist, welches Folgendes umfasst:

i. einen anti-BDNF-Fangantikörper und einen anti-CA125-Fangantikörper;
ii. einen anti-BDNF-Detektionsantikörper und einen anti-CA125-Detektionsantikörper, wobei der Detektionsantikörper mit einem Detektions-Tag, wie einem Enzym (zum Beispiel, Peroxidase oder alkalischer Phosphatase) oder Biotin, konjugiert ist;
iii. ein Enzymsubstrat, wie Tetramethylbenzidin (TMBD);
iv. wenn der Detektionsantikörper mit Biotin konjugiert ist, ein enzymkonjugiertes Streptavidin;
v. wahlweise, einen rekombinanten menschlichen BDNF und einen rekombinanten menschlichen CA125 für dem Kalibrator und die Positivkontrollen;
vi. wahlweise, zusätzlich umfassend Anweisungen für die Verwendung der genannten Reagenzien bei der Bestimmung der Proteinniveaus von BDNF und CA125 in einer Serumprobe, welche aus einem weiblichen Individuum isoliert wurde.

**Revendications**

1. Procédé de dépistage, de diagnostic ou de surveillance de l'endométriose ou de la présence de lésions de type endométriose chez un sujet féminin, dans lequel ledit procédé comprend :

a) déterminer les niveaux protéiques de :

i. antigène 125 du cancer (CA125) dans un échantillon de sang, de plasma ou de sérum isolé du sujet, et
ii. facteur neurotrophique dérivé du cerveau (BDNF) dans un échantillon de sérum isolé du sujet ; et

b) optionnellement, calculer un score à partir des niveaux protéiques déterminés dans l'étape a), optionnellement en combinaison avec d'autres marqueurs génétiques ou protéiques, des paramètres biochimiques et/ou des variables liées à l'histoire et/ou aux caractéristiques cliniques du sujet ;
c) comparer les niveaux protéiques de CA125 et de BDNF dans l'échantillon du sujet tels que déterminés dans l'étape a) avec une valeur de référence correspondante ; ou comparer le score obtenu dans b) avec une valeur de référence ; dans lequel, lorsque les niveaux protéiques de CA125 et BDNF dans a), ou le score dans b), sont augmentés par rapport à la valeur de référence respective, cela est indicatif d'endométriose ou de la présence de lésions de type endométriose.

2. Procédé selon la revendication 1, dans lequel ledit sujet est caractérisé ou a été présélectionnée pour avoir des signes et/ou des symptômes de l'endométriose, de préférence pour avoir une douleur pelvienne chronique et/ou infertilité.

3. Procédé selon l'une quelconque des revendications 1 ou 2, dans lequel ledit sujet n'est pas enceinte, ni souffre d'aucune autre maladie inflammatoire.

4. Procédé selon l'une quelconque des revendications 1 à 3, dans lequel les niveaux protéiques de BDNF comprennent des niveaux de proBDNF et de BDNF mature (mBDNF), les niveaux de BDNF dits totaux.

5. Procédé selon l'une quelconque des revendications 1 à 4, dans lequel la détermination des niveaux de BDNF et de CA125 est mise en œuvre par le biais d'un immunoessai, de préférence un essai d'immunoadsorption par enzyme liée (ELISA), un essai d'immunoadsorption fluorescent (FIA), un immunoessai de chimioluminescence (CIA), ou un radioimmunoessai (RIA), un immunoessai à multiplication enzymatique, un radioimmunoessai en phase solide (SPROA), un essai de polarisation de fluorescence (FP), un essai de transfert d'énergie par résonance de fluorescence (FRET), un essai de transfert d'énergie par résonance de fluorescence à résolution dans le temps (TR-FRET), un essai de résonance plasmonique de surface (SPR), y compris toutes les versions multiplex de l'un quelconque de ceux-ci.

6. Procédé selon l'une quelconque des revendications 1 à 5, dans lequel ledit immunoessai est un essai ELISA ou toute version multiplex de celui-ci.

**7.** Procédé selon la revendication 6, dans lequel ledit essai ELISA est un test ELISA en sandwich et comprend :

  a. un anticorps de capture ; et
  b. un anticorps de détection, dans lequel l'anticorps de détection est conjugué à une étiquette de détection, telle qu'une enzyme ou la biotine.

**8.** Procédé selon l'une quelconque des revendications 1 à 7, dans lequel, pour le calcul du score dans l'étape b), les niveaux protéiques de BDNF et de CA125 sont utilisés comme variables, en combinaison avec une, deux, trois, quatre, cinq, six, sept ou huit des variables liées aux caractéristiques du sujet choisies dans le groupe consistant en :

  a) une histoire de chirurgie pour le diagnostic de l'endométriose ;
  b) des menstruations douloureuses comme symptôme pour l'examen de l'endométriose ;
  c) l'utilisation d'implant hormonal ; et
  d) la fréquence de selles plus dures dans le cas de douleur menstruelle dans les 3 derniers mois ;
  e) l'âge au moment de la première expérience de douleur pendant des rapports sexuels ;
  f) l'âge au moment de la première expérience de symptômes de kyste de l'ovaire ;
  g) l'intensité de la douleur menstruelle la dernière fois (1-10) ; et
  h) l'âge de début d'utilisation d'ibuprofène.

**9.** Procédé selon la revendication 8, dans lequel lesdites variables sont une, deux, trois ou quatre choisies dans le groupe consistant en :

  a) une histoire de chirurgie pour le diagnostic de l'endométriose ;
  b) des menstruations douloureuses comme symptôme pour l'examen de l'endométriose ;
  c) l'utilisation d'implant hormonal ; et
  d) la fréquence de selles plus dures dans le cas de douleur menstruelle dans les 3 derniers mois.

**10.** Procédé selon l'une quelconque des revendications 1 à 9, dans lequel ledit sujet est un sujet féminin humain.

**11.** Procédé selon l'une quelconque des revendications 1 à 10, dans lequel ledit procédé comprend en outre la réalisation d'un test de confirmation, tel qu'une confirmation histologique après laparoscopie.

**12.** Procédé mis en œuvre par ordinateur (CI) de dépistage, de diagnostic ou de surveillance de l'endométriose chez un sujet, de préférence un sujet féminin, dans lequel ledit procédé comprend :

  i. recevoir par un programme d'ordinateur des informations sur les niveaux protéiques d'un groupe de biomarqueurs dans un échantillon de sérum isolé du sujet, dans lequel lesdits biomarqueurs consistent en BDNF et en CA125, et optionnellement des informations sur d'autres marqueurs génétiques ou protéiques, des paramètres biochimiques et/ou des variables liées à l'histoire et/ou aux caractéristiques cliniques du sujet ;
  ii. traiter les informations reçues selon l'étape i) par la réalisation de l'étape c), et optionnellement de l'étape b), selon l'une quelconque des revendications 1 à 11 ;
  iii. générer des informations de sortie, par exemple à travers un affichage de terminal, identifiant ou classifiant un sujet ayant ou à forte probabilité d'avoir de l'endométriose ou comme un individu pouvant bénéficier d'un test exploratoire, tel qu'une laparoscopie.

**13.** Programme d'ordinateur comprenant des instructions qui, lorsque le programme est exécuté par un ordinateur, amènent l'ordinateur à mettre en oeuvre les étapes ii) et/ou iii), du procédé selon la revendication 12.

**14.** Appareil de traitement de données comprenant des moyens pour mettre en œuvre les étapes ii) et/ou iii) du procédé selon la revendication 12.

**15.** Utilisation *in vitro* d'un kit approprié pour déterminer les niveaux protéiques de BDNF et de CA125 dans un échantillon de sérum isolé d'un sujet, dans un procédé de dépistage, de diagnostic ou de surveillance de l'endométriose ou de la présence de lésions de type endométriose chez un sujet selon l'une quelconque des revendications 1 à 11 ; dans lequel ledit kit comprend :

  i. un réactif pour la quantification des niveaux protéiques de BDNF ;
  ii. un réactif pour la quantification des niveaux protéiques de CA125 ;

iii. optionnellement, un BDNF humain recombinant et un CA125 humain recombinant pour l'étalonneur et les témoins positifs ;

iv. optionnellement, comprenant en outre des instructions pour l'utilisation desdits réactifs dans la détermination des niveaux protéiques de BDNF et de CA125 dans un échantillon de sérum isolé d'un sujet.

16. Utilisation d'un kit selon la revendication 15, dans lequel ledit kit est un kit d'ELISA en sandwich qui comprend :

i. un anticorps de capture anti-BDNF et un anticorps de capture anti-CA125 ;

ii. un anticorps de détection anti-BDNF et un anticorps de détection anti-CA125, dans lequel l'anticorps de détection est conjugué à une étiquette de détection, telle qu'une enzyme (par exemple, la peroxydase ou la phosphatase alcaline) ou la biotine ;

iii. un substrat enzymatique, tel que la tétraméthylbenzidine (TMBD) ;

iv. lorsque l'anticorps de détection est conjugué à la biotine, une streptavidine conjugué à une enzyme ;

v. optionnellement, un BDNF humain recombinant et un CA125 humain recombinant pour l'étalonneur et les témoins positifs ;

vi. optionnellement, comprenant en outre des instructions pour l'utilisation desdits réactifs dans la détermination des niveaux protéiques de BDNF et de CA125 dans un échantillon de sérum isolé d'un sujet féminin.

**FIG. 1**

| | All | Cases | Controls |
|---|---|---|---|
| **General** | | | |
| **Sample size** | 205 | 137 | 68 |
| **Missing values** | 1 | 1 | 0 |
| **Range** | 1.54 - 796 | 2.04 - 166 | 1.54 - 796 |
| **Mean and dispersion around mean** | | | |
| **Mean** | 25.2 | 25.8 | 24.1 |
| **SEM** | 4.46 | 2.86 | 12.2 |
| **CI95 of mean** | 16.5 - 34.0 | 20.2 - 31.4 | 0.298 - 48.0 |
| **Standard deviation** | 63.7 | 33.3 | 100 |
| **Median and dispersion around median** | | | |
| **Median** | 10.1 | 12.1 | 7.14 |
| **CI95 of median** | 9.17 - 11.4 | 10.2 - 14.0 | 6.45 - 8.93 |
| **Interquartile range** | 6.35 - 17.0 | 7.43 - 27.7 | 5.71 - 11.3 |
| **Others** | | | |
| **Shapiro-Wilk $W$** | 0.289 | 0.652 | 0.175 |
| **Shapiro-Wilk $p$** | 2.12E-27 | 1.68E-16 | 1.16E-17 |
| **Kurtosis** | 103 | 5.29 | 49.1 |
| **Skewness** | 9.18 | 2.36 | 6.91 |

**FIG.2**

| | All | Cases | Controls |
|---|---|---|---|
| **General** | | | |
| Sample size | 205 | 137 | 68 |
| Missing values | 2 | 1 | 1 |
| Range | 1.27 - 801 | 1.27 - 184 | 1.64 - 801 |
| **Mean and dispersion around mean** | | | |
| Mean | 26.5 | 27.3 | 25 |
| SEM | 4.56 | 3.02 | 12.4 |
| CI95 of mean | 17.6 - 35.5 | 21.4 - 33.2 | 0.577 - 49.4 |
| Standard deviation | 65 | 35.3 | 102 |
| **Median and dispersion around median** | | | |
| Median | 10.9 | 12.8 | 7.38 |
| CI95 of median | 9.66 - 12.3 | 11.4 - 14.6 | 6.47 - 8.54 |
| Interquartile range | 6.50 - 19.0 | 7.68 - 29.3 | 5.70 - 11.9 |
| **Others** | | | |
| Shapiro-Wilk *W* | 0.304 | 0.656 | 0.181 |
| Shapiro-Wilk *p* | 4.12E-27 | 2.11E-16 | 1.79E-17 |
| Kurtosis | 97.9 | 5.48 | 47.8 |
| Skewness | 8.87 | 2.37 | 6.82 |

FIG. 3

Plasma Glycodelin (ng/mL)

| | All | Cases | Controls |
|---|---|---|---|
| **General** | | | |
| Sample size | 205 | 137 | 68 |
| Missing values | 3 | 3 | 0 |
| Range | 1.50 - 202 | 1.50 - 202 | 1.50 - 93.9 |
| **Mean and dispersion around mean** | | | |
| Mean | 20.2 | 22.2 | 16 |
| SEM | 2.08 | 2.86 | 2.47 |
| CI95 of mean | 16.1 - 24.2 | 16.6 - 27.8 | 11.2 - 20.9 |
| Standard deviation | 29.5 | 33.1 | 20.4 |
| **Median and dispersion around median** | | | |
| Median | 8.8 | 9.09 | 7.06 |
| CI95 of median | 6.12 - 10.4 | 6.97 - 11.9 | 3.38 - 14.0 |
| Interquartile range | 1.50 - 23.8 | 3.50 - 25.0 | 1.50 - 21.7 |
| **Others** | | | |
| Shapiro-Wilk *W* | 0.655 | 0.648 | 0.744 |
| Shapiro-Wilk *p* | 4.71E-20 | 1.84E-16 | 1.50E-09 |
| Kurtosis | 11 | 9.31 | 2.65 |
| Skewness | 2.89 | 2.77 | 1.74 |

**FIG. 4**

Serum Glycodelin (ng/mL)

| | All | Cases | Controls |
|---|---|---|---|
| **General** | | | |
| Sample size | 205 | 137 | 68 |
| Missing values | 8 | 7 | 1 |
| Range | 1.50 - 171 | 1.50 - 171 | 1.50 - 60.3 |
| **Mean and dispersion around mean** | | | |
| Mean | 12.7 | 13.2 | 11.7 |
| SEM | 1.4 | 1.9 | 1.82 |
| CI95 of mean | 9.91 - 15.4 | 9.42 - 16.9 | 8.11 - 15.3 |
| Standard deviation | 19.6 | 21.7 | 14.9 |
| **Median and dispersion around median** | | | |
| Median | 5.28 | 5.3 | 4.87 |
| CI95 of median | 3.92 - 6.57 | 3.86 - 6.46 | 1.50 - 9.70 |
| Interquartile range | 1.50 - 15.4 | 1.50 - 14.0 | 1.50 - 16.6 |
| **Others** | | | |
| Shapiro-Wilk *W* | 0.596 | 0.564 | 0.714 |
| Shapiro-Wilk *p* | 2.84E-21 | 5.54E-18 | 4.09E-10 |
| Kurtosis | 21.9 | 21.3 | 2.82 |
| Skewness | 3.8 | 3.92 | 1.85 |

**FIG. 5**

| | **All** | **Cases** | **Controls** |
|---|---|---|---|
| **General** | | | |
| Sample size | 205 | 137 | 68 |
| Missing values | 1 | 1 | 0 |
| Range | 312 - 17400 | 312 - 16000 | 312 - 17400 |
| **Mean and dispersion around mean** | | | |
| Mean | 2200 | 2150 | 2290 |
| SEM | 199 | 239 | 359 |
| CI95 of mean | 1810 - 2590 | 1680 - 2620 | 1580 - 2990 |
| Standard deviation | 2840 | 2790 | 2960 |
| **Median and dispersion around median** | | | |
| Median | 1240 | 1220 | 1300 |
| CI95 of median | 1050 - 1500 | 1050 - 1520 | 625 - 1890 |
| Interquartile range | 625 - 2620 | 312 - 2570 | 625 - 2790 |
| **Others** | | | |
| Shapiro-Wilk *W* | 0.639 | 0.631 | 0.642 |
| Shapiro-Wilk *p* | 1.47E-20 | 5.48E-17 | 1.35E-11 |
| Kurtosis | 10.3 | 10.4 | 9.65 |
| Skewness | 2.99 | 3.03 | 2.86 |

**FIG. 6**

| | All | Cases | Controls |
|---|---|---|---|
| **General** | | | |
| Sample size | 205 | 137 | 68 |
| Missing values | 1 | 1 | 0 |
| Range | 5490 - 39600 | 8300 - 39600 | 5490 - 37600 |
| **Mean and dispersion around mean** | | | |
| Mean | 21100 | 21700 | 20100 |
| SEM | 366 | 441 | 637 |
| CI95 of mean | 20400 - 21900 | 20800 - 22500 | 18800 - 21300 |
| Standard deviation | 5220 | 5140 | 5250 |
| **Median and dispersion around median** | | | |
| Median | 21000 | 21800 | 19200 |
| CI95 of median | 19800 - 22200 | 20300 - 22600 | 17900 - 21300 |
| Interquartile range | 17500 - 24300 | 18600 - 25000 | 16700 - 23600 |
| **Others** | | | |
| Shapiro-Wilk *W* | 0.99 | 0.99 | 0.977 |
| Shapiro-Wilk *p* | 0.177 | 0.424 | 0.235 |
| Kurtosis | 0.511 | 0.243 | 1.04 |
| Skewness | 0.309 | 0.302 | 0.372 |

**FIG. 7**

| | All | Cases | Controls |
|---|---|---|---|
| **General** | | | |
| Sample size | 205 | 137 | 68 |
| Missing values | 1 | 1 | 0 |
| Range | 20100 - 66200 | 20100 - 66200 | 28000 - 63000 |
| **Mean and dispersion around mean** | | | |
| Mean | 37700 | 37700 | 37600 |
| SEM | 497 | 648 | 745 |
| CI95 of mean | 36700 - 38600 | 36500 - 39000 | 36100 - 39000 |
| Standard deviation | 7100 | 7550 | 6150 |
| **Median and dispersion around median** | | | |
| Median | 37400 | 37700 | 37000 |
| CI95 of median | 36200 - 38000 | 35200 - 38600 | 36100 - 38100 |
| Interquartile range | 33600 - 41000 | 33400 - 41800 | 34100 - 39800 |
| **Others** | | | |
| Shapiro-Wilk *W* | 0.958 | 0.965 | 0.92 |
| Shapiro-Wilk *p* | 1.10E-05 | 0.00158 | 0.000328 |
| Kurtosis | 2.09 | 1.68 | 2.83 |
| Skewness | 0.833 | 0.717 | 1.17 |

**FIG. 8**

Serum ZAG (ng/mL)

| | All | Cases | Controls |
|---|---|---|---|
| **General** | | | |
| Sample size | 205 | 137 | 68 |
| Missing values | 1 | 1 | 0 |
| Range | 18700 - 51800 | 18700 - 51800 | 22400 - 50900 |
| **Mean and dispersion around mean** | | | |
| Mean | 32600 | 32300 | 33200 |
| SEM | 376 | 474 | 610 |
| CI95 of mean | 31800 - 33300 | 31300 - 33200 | 32000 - 34400 |
| Standard deviation | 5380 | 5530 | 5030 |
| **Median and dispersion around median** | | | |
| Median | 32000 | 31600 | 32000 |
| CI95 of median | 31100 - 33400 | 30600 - 33400 | 31200 - 34000 |
| Interquartile range | 29600 - 35400 | 28700 - 35600 | 30000 - 35400 |
| **Others** | | | |
| Shapiro-Wilk $W$ | 0.978 | 0.985 | 0.947 |
| Shapiro-Wilk $p$ | 0.00298 | 0.153 | 0.00628 |
| Kurtosis | 1.03 | 0.715 | 1.56 |
| Skewness | 0.538 | 0.42 | 0.932 |

**FIG.9**

Serum CA125 = 0.79 + 1.02 x Plasma CA125

**FIG.10**

Serum Glycodelin = 2.28 + 0.52 x Plasma Glycodelin

FIG. 11

Serum Total BDNF = 20711.75 + 0.19 x Plasma Total BD

FIG.12

Serum ZAG = 12627.95 + 0.53 x Plasma ZAG

**FIG.13**

**A)**

CA125

**B)**

BDNF

**FIG.14**

| Gene ID | Symbol | Transcript | Length (nt) | Protein | Length (aa) | Protein name | Isoform | Organism |
|---|---|---|---|---|---|---|---|---|
| 627 | BDNF | NM_170731.5 | 4312 | NP_733927.1 | 255 | brain-derived neurotrophic factor | b precursor | Homo sapiens |
| 627 | BDNF | NM_00114380 5.1 | 3827 | NP_0011372 77.1 | 247 | brain-derived neurotrophic factor | a preproprotein | Homo sapiens |
| 627 | BDNF | NM_00114380 6.1 | 4042 | NP_0011372 78.1 | 247 | brain-derived neurotrophic factor | a preproprotein | Homo sapiens |
| 627 | BDNF | NM_170732.4 | 4125 | NP_733928.1 | 247 | brain-derived neurotrophic factor | a preproprotein | Homo sapiens |
| 627 | BDNF | NM_00114380 7.2 | 3749 | NP_0011372 79.1 | 247 | brain-derived neurotrophic factor | a preproprotein | Homo sapiens |
| 627 | BDNF | NM_170733.4 | 4018 | NP_733929.1 | 247 | brain-derived neurotrophic factor | a preproprotein | Homo sapiens |
| 627 | BDNF | NM_00114380 8.2 | 3766 | NP_0011372 80.1 | 247 | brain-derived neurotrophic factor | a preproprotein | Homo sapiens |
| 627 | BDNF | NM_00114380 9.2 | 3776 | NP_0011372 81.1 | 276 | brain-derived neurotrophic factor | d | Homo sapiens |
| 627 | BDNF | NM_00114381 0.2 | 4059 | NP_0011372 82.1 | 329 | brain-derived neurotrophic factor | e | Homo sapiens |
| 627 | BDNF | NM_00114381 1.2 | 4176 | NP_0011372 83.1 | 247 | brain-derived neurotrophic factor | a preproprotein | Homo sapiens |
| 627 | BDNF | NM_00114381 2.2 | 3918 | NP_0011372 84.1 | 247 | brain-derived neurotrophic factor | a preproprotein | Homo sapiens |
| 627 | BDNF | NM_00114381 3.2 | 3967 | NP_0011372 85.1 | 247 | brain-derived neurotrophic factor | a preproprotein | Homo sapiens |
| 627 | BDNF | NM_001709.5 | 3985 | NP_001700.2 | 247 | brain-derived neurotrophic factor | a preproprotein | Homo sapiens |
| 627 | BDNF | NM_00114381 4.2 | 4092 | NP_0011372 86.1 | 247 | brain-derived neurotrophic factor | a preproprotein | Homo sapiens |
| 627 | BDNF | NM_170734.4 | 3979 | NP_733930.1 | 262 | brain-derived neurotrophic factor | c | Homo sapiens |
| 627 | BDNF | NM_00114381 6.2 | 4532 | NP_0011372 88.1 | 247 | brain-derived neurotrophic factor | a preproprotein | Homo sapiens |
| 627 | BDNF | NM_170735.6 | 4766 | NP_733931.1 | 247 | brain-derived neurotrophic factor | a preproprotein | Homo sapiens |

**FIG.15**

| Gene ID | Symbol | Transcript | Length (nt) | Protein | Length (aa) | Protein name | Isoform | Organism |
|---|---|---|---|---|---|---|---|---|
| 94025 | MUC16 | NM_001401501.1 | 46219 | NP_001388430.1 | 15349 | mucin-16 precursor | | Homo sapiens |
| 94025 | MUC16 | NM_024690.2 | 43816 | NP_078966.2 | 14507 | mucin-16 | | Homo sapiens |

FIG.16

EP 4 616 199 B1

MLKPSGLPGSSSPTRSLMTGSRSTKATPEMDSGLTGATLSPKTSTGAIVVTEHTLPFTSPDKTLASPTSSVVGRTTQSLGVMSSALPESTSRGMTHSEQRTSPSLSPQVNGTPSRNYPATSMVSGLSSPRTRTSSTEGNFTK
EASTYTLTVETTSGPVTEKYTVPTETSTTEGDSTETPWDTRYIPVKITSPMKTFADSTASKENAPVSMTPAETTVTDSHTPGRTNPSFGTLYSSFLDLSPKGTPNSRGETSLELILSTTGYPFSSPEPGSAGHSRISTSAPLSSS
ASVLDNKISETSIFSGQSLTSPLSPGVPEARASTMPNSAIPFSMTLSNAETSAERVRSTISSLGTPSISTKQTAETILTFHAFAETMDIPSTHIAKTLASEWLGSPGTLGGTSTSALTTTSPSTTLVSEETNTHHSTSGKETEGTL
NTSMTPLETSAPGEESEMTATLVPTLGFTTLDSKIRSPSQVSSSHPTRELRTTGSTSGRQSSSTAAHGSSDILRATTSSTSKASSWTSESTAQQFSEPQHTQWVETSPSMKTERPPASTSVAAPITTSVPSVVSGFTTLKTSS
TKGIWLEETSADTLIGESTAGPTTHQFAVPTGISMTGGSSTRGSQGTTHLLTRATASSETSADLTLATNGVPVSVSPAVSKTAAGSSPPGGTKPSYTMVSSVIPETSSLQSSAFREGTSLGLTPLNTRHPFSSPEPDSAGHTKI
STSIPLLSSASVLEDKVSATSTFSHHKATSSITTGTPEISTKTKPSSAVLSSMTLSNAATSPERVRNATSPLTHPSPSGEETAGSVLTLSTSAETTDSPNIHPTGTLTSESSESPSTLSLPSVSGVKTTFSSSTPSTHLFTSGEETEET
SNPSVSQPETSVSRVRTTLASTSVPTPVFPTMDTWPTRSAQFSSSHLVSELRATSSTSVTNSTGSALPKISHLTGTATMSQTNRDTFNDSAAPQSTTWPETSPRFKTGLPSATTTVSTSATSLSATVMVSKFTSPATSSMEA
TSIREPSTTILTTETTNGPGSMAVASTNIPIGKGYITEGRLDTSHLPIGTTASSETSMDFTMAKESVSMSVSPSQSMDAAGSSTPGRTSQFVDTFSDDVYHLTSREITIPRDGTSSALTPQMTATHPPSPDPGSARSTWLGIL
SSSPSSPTPKVTMSSTFSTQRVTTSMIMDTVETSRWNMPNLPSTTSLTPSNIPTSGAIGKSTLVPLDTPSPATSLEASEGGLPTLSTYPESTNTPSIHLGAHASSESPSTIKLTMASVVKPGSYTPLTFPSIETHIHVSTARMAY
SSGSSPEMTAPGETNTGSTWDPTTYITTTDPKDTSSAQVSTPHSVRTLRTTENHPKTESATPAAYSGSPKISSSPNLTSPATKAWTITDTTEHSTQLHYTKLAEKSSGFETQSAPGPVSVVIPTSPTIGSSTLELTSDVPGEPL
VLAPSEQTTITLPMATWLSTSLTEEMASTDLDISSPSSPMSTFAIFPPMSTPSHELSKSEADTSAIRNTDSTTLDQHLGIRSLGRTGDLTTVPITPLTTTWTSVIEHSTQAQDTLSATMSPTHVTQSLKDQTSIPASASPSHLTE
VYPELGTQGRSSSEATTFWKPSTDTLSREIETGPTNIQSTPPMDNTTTGSSSSGVTLGIAHLPIGTSSPAETSTNMALERRSSTATVSMAGTMGLLVTSAPGRSISQSLGRVSSVLSESTTEGVTDSSKGSSPRLNTQGNTAL
SSSLEPSYAEGSQMSTSIPLTSSPTTPDVEFIGGSTFWTKEVTTVMTSDISKSSARTESSSATLMSTALGSTENTGKEKLRTASMDLPSPTPSMEVTPWISLTLSNAPNTTDSLDLSHGVHTSSAGTLATDRSLNTGVTRASR
LENGSDTSSKSLSMGNSTHTSMTYTEKSEVSSSIHPRPETSAPGAETTLTSTPGNRAISLTLPFSSIPVEEVISTGITSGPDINSAPMTHSPITPPTIVWTSTGTIEQSTQPLHAVSSEKVSVQTQSTPYVNSVAVSASPTHENS
VSSGSSTSSPYSSASLESLDSTISRRNAITSWLWDLTTSLPTTTWPSTSLSEALSSGHSGVSNPSSTTTEFPLFSAASTSAAKQRNPETETHGPQNTAASTLNTDASSVTGLSETPVGASISSEVPLPMAITSRSDVSGLTSEST
ANPSLGTASSAGTKLTRTISLPTSESLVSFRMNKDPWTVSIPLGSHPTTNTETSIPVNSAGPPGLSTVASDVIDTPSDGAESIPTVSFSPSPDTEVTTISHFPEKTTHSFRTISSLTHELTSRVTPIPGDWMSSAMSTKPTGASP
SITLGERRTITSAAPTTSPIVLTASFTETSTVSLDNETTVKTSDILDARKTNELPSDSSSSSDLINTSIASSTMDVTKTASISPTSISGMTASSSPSLFSSDRPQVPTSTTETNTATSPSVSSNTYSLDGGSNVGGTPSTLPPFTITH
PVETSSALLAWSRPVRTFSTMVSTDTASGENPTSSNSVVTSVPAPGTWTSVGSTTDLPAMGFLKTSPAGEAHSLLASTIEPATAFTPHLSAAVVTGSSATSEASLLTTSESKAIHSSPQTPTTPTSGANWETSATPESLLVVT
ETSDTTLTSKILVTDTILFSTVSTPPSKFPSTGTLSGASFPTLLPDTPAIPLTATEPTSSLATSFDSTPLVTIASDSLGTVPETTLTMSETSNGDALVLKTVSNPDRSIPGITIQGVTESPLHPSSTSPSKIVAPRNTTYEGSITVALST
LPAGTTGSLVFSQSSENSETTALVDSSAGLERASVMPLTTGSQGMASSGGIRSGSTHSTGTKTFSSLPLTMNPGEVTAMSEITTNRLTATQSTAPKGIPVKPTSAESGLLTPVSASSSPSKAFASLTTAPPTWGIPQSTLTFEFS
EVPSLDTKSASLPTPGQSLNTIPDSDASTASSSLSKSPEKNPRARMMTSTKAISASSFQSTGFTETPEGSASPSMAGHEPRVPTSGTGDPRYASESMSYPDPSKASSAMTSTSLASKLTTLFSTGQAARSGSSSSPISLSTEK
ETSFLSPTASTSRKTSLFLGPSMARQPNILVHLQTSALTLSPTSTLNMSQEEPPELTSSQTIAEEEGTTAETQTLTFTPSETPTSLLPVSSPTEPTARRKSSPETWASSISVPAKTSLVETTDGTLVTTIKMSSQAAQGNSTWPA
PAEETGSSPAGTSPGSPEMSTTLKIMSSKEPSISPEIRSTVRNSPWKTPETTVPMETTVEPVTLQSTALGSGSTSISHLPTGTTSPTKSPTENMLATERVSLSPSPPEAWTNLYSGTPGGTRQSLATMSSVSLESPTARSITGT
GQQSSPELVSKTTGMEFSMWHGSTGGTTGDTHVSLSTSSNILEDPVTSPNSVSSLTDKSKHKTETWVSTTAIPSTVLNNKIMAAEQQTSRSVDEAYSSTSSWSDQTSGSDITLGASPDVTNTLYITSTAQTTSLVSLPSGD
QGITSLTNPSGGKTSSASSVTSPSIGLETLRANVSAVKSDIAPTAGHLSQTSSPAEVSILDVTTAPTPGISTTITTMGTNSISTTTPNPEVGMSTMDSTPATERRTTSTEHPSTWSSTAASDSWTVTDMTSNLKVARSPGTIS
TMHTTSFLASSTELDSMSTPHGRITVIGTSLVTPSSDASAVKTETSTSERTLSPSDTTASTPISTFSRVQRMSISVPDILSTSWTPSSTEAEDVPVSMVSTDHASTKTDPNTPLSTFLFDSLSTLDWDTGRSLSSATATTSAPQ
GATTPQELTLETMISPATSQLPFSIGHITSAVTPAAMARSSGVTFSRPDPTSKKAEQTSTQLPTTTSAHPGQVPRSAATTLDVIPHTAKTPDATFQRQGQTALTTEARATSDSWNEKEKSTPSAPWITEMMNSVSEDTIKE
VTSSSSVLRTLNTLDINLESGTTSSPSWKSSPYERIAPSESTTDKEAIHPSTNTVETTGWVTSSEHASHSTIPAHSASSKLTSPVVTTSTREQAIVSMSTTTWPESTRARTEPNSFLTIELRDVSPYMDTSSTTQTSIISSPGSTA
ITKGPRTEITSSKRISSSFLAQSMRSSDSPSEAITRLSNFPAMTESGGMILAMQTSPPGATSLSAPTLDTSATASWTGTPLATTQRFTYSEKTTLFSKGPEDTSQPSPPSVEETSSSSSLVPIHATTSPSNILLTSQGHSPSSTPP
VTSVFLSETSGLGKTTDMSRISLEPGTSLPPNLSSTAGEALSTYEASRDTKAIHHSADTAVTNMEATSSEYSPIPGHTKPSKATSPLVTSHIMGDITSSTSVFGSSETTEIETVSSVNQGLQERSTSQVASSATETSTVITHVSS
GDATTHVTKTQATFSSGTSISSPHQFITSTNTFTDVSTNPSTSLIMTESSGVTITTQTGPTGAATQGPYLLDTSTMPYLTETPLAVTPDFMQSEKTTLISKGPKDVSWTSPPSVAETSYPSSLTPFLVTTIPPATSTLQGQHTS
SPVSATSVLTSGLVKTTDMLNTSMEPVTNSPQNLNNPSNEILATLAATTDIETIHPSINKAVTNMGTASSAHVLHSTLPVSSEPSTATSPMVPASSMGDALASISIPGSETTDIEGEPTSSLTAGRKENSTLQEMNSTTESNII
LSNVSVGAITEATKMEVPSFDATFIPTPAQSTKFPDIFSVASSRLSNSPPMTISTHMTTTQTGSSGATSKIPLALDTSTLETSAGTPSVVTEGFAHSKITTAMNNDVKDVSQTNPPFQDEASSPSSQAPVLVTTLPSSVAFTP

## REFERENCES CITED IN THE DESCRIPTION

**Patent documents cited in the description**

- WO 2015051451 A
- US 20180067102 A
- US 20100173786 A
- WO 2012155019 A
- US 20110039287 A

**Non-patent literature cited in the description**

- *Nat Rev Endocrinol.*, November 2019, vol. 15 (11), 666-682
- **LEE SY** ; **KOO YJ** ; **LEE DH.** Classification of endometriosis. *Yeungnam Univ J Med.*, January 2021, vol. 38 (1), 10-18
- Revised American Society for Reproductive Medicine classification of endometriosis. *Fertil Steril.*, 1996, vol. 67 (5), 817-21
- **WEIDLE et al.** *Cancer Genomics & Proteomics*, 2013, vol. 10, 1-18
- **LOFBLOM, J. et al.** *Curr. Opin. Biotechnol.*, 2011, vol. 22, 843-848
- **BANTA, S. et al.** *Annu. Rev. Biomed. Eng.*, 2010, vol. 15, 93-113
- **MIHM M** ; **GANGOOLY S** ; **MUTTUKRISHNA S**. The normal menstrual cycle in women. *Anim Reprod Sci.*, April 2011, vol. 124 (3-4), 229-36
- **M. RAUH**. *J Chromatogr B Analyt Technol Biomed Life Sci*, 01 February 2012, vol. 883-884, 59-67
- **JOHNSON N.P.** Consensus on current Management of endometriosis. *Hum. Reprod*, 2013, vol. 28 (6), 1552-1568
- **ZONDERVAN, K.T.** Endometriosis. *Nat. Rev. Dis. Primer*, 2018, vol. 4, 9
- **ZONDERVAN, K.T.** Endometriosis. *N. Engl. J. Med*, 2020, vol. 382, 1244-1256
- **VIGANO P.** Endometriosis: epidemiology and aetiological factors.. *Best Pract. Res. Clin. Obstet. Gynaecol*, 2004, vol. 18, 177-200
- **SOMIGLIANA E.** Association between endometriosis and cancer: a comprehensive review and a critical analysis of clinical and epidemiological evidence.. *Gynecol. Oncol*, 2006, vol. 101 (2), 331-341
- **SINAII N.** High rates of autoimmune and endocrine disorders, fibromyalgia, chronic fatigue syndrome and atopic diseases among women with endometriosis: a survey analysis.. *Hum. Reprod*, 2002, vol. 17 (10), 2715-2724
- **AGARWAL, S. K.** ; **CHAPRON, C.** ; **GIUDICE, L. C.** ; **LAUFER, M. R.** ; **LEYLAND, N.** ; **MISSMER, S. A.** ; **TAYLOR, H. S.** Clinical diagnosis of endometriosis: a call to action.. *American journal of obstetrics and gynecology*, 2019, vol. 220 (4), 354-e1
- **BECKER, C.** ESHRE guideline: endometriosis.. *Human Reproduction Open*, 2022, vol. 2, 1-26
- **DUNSELMAN, G.J.** ESHRE guideline: management of women with endometriosis. *Hum. Reprod. Oxf. Engl*, 2014, vol. 29, 400-412
- Randomized controlled trial of depot leuprolide in patients with chronic pelvic pain and clinically suspected endometriosis. **LING, F.W.** Obstet. Gynecol. Pelvic Pain Study Group, 1999, vol. 93, 51-58
- **JENKINS, T.R**. Does response to hormonal therapy predict presence or absence of endometriosis. *J. Minim. Invasive Gynecol*, 2008, vol. 15, 82-86
- **AHN SH** ; **SINGH V** ; **TAYADE C.** Biomarkers in endometriosis: challenges and opportunities. *Fertil Steril*, 2017, vol. 107 (3), 523-532
- **NISENBLAT, V.** Combination of the non-invasive tests for the diagnosis of endometriosis. *Cochrane Database Syst. Rev.*, 2016, vol. 7, CD012281
- **NISENBLAT, V.** Blood biomarkers for the non-invasive diagnosis of endometriosis. *Cochrane Database Syst. Rev.*, 2016, vol. 5 (CD012179)
- **POLACCHINI A et al.** A method for reproducible measurements of serum BDNF: comparison of the performance of six commercial assays. *Sci Rep.*, 10 December 2015, vol. 5, 17989
- **KECKSTEIN J** ; **SARIDOGAN E** ; **ULRICH UA** ; **SILLEM M** ; **OPPELT P** ; **SCHWEPPE KW** ; **KRENTEL H** ; **JANSCHEK E** ; **EXACOUSTOS C** ; **MALZONI M**. The #Enzian classification: A comprehensive non-invasive and surgical description system for endometriosis. *Acta Obstet Gynecol Scand.*, July 2021, vol. 100 (7), 1165-1175
- **CAPEZZUOLI et al.** Classification/staging systems for endometriosis: the state of the art. *Gynecological and Reproductive Endocrinology and Metabolism*, 2020, vol. 1 (1), 14-22

- **KITAWAKI J** ; **ISHIHARA H** ; **KOSHIBA H** ; **KIYOMIZU M** ; **TERAMOTO M** ; **KITAOKA Y** ; **HONJO H.** Usefulness and limits of CA-125 in diagnosis of endometriosis without associated ovarian endometriomas. *Hum Reprod.*, July 2005, vol. 20 (7), 1999-2003

- **BARBIERI RL** ; **NILOFF JM** ; **BAST RC JR** ; **SCAETZL E** ; **KISTNER RW** ; **KNAPP RC**. Elevated serum concentrations of CA-125 in patients with advanced endometriosis. *Fertil Steril*, 1986, vol. 45, 630-634